# EUROPEAN PATENT APPLICATION

(11) **EP 4 342 980 A1**
(43) Date of publication of application: **27.03.2024**
(21) Application number: 22804750.2
(22) Date of filing: 19.05.2022
(51) Int. Cl.: C12N 5/071, C12N 5/074, C12Q 1/02, C07K 14/50, C07K 14/78

(54) **METHOD FOR PRODUCING BRAIN CAPILLARY ENDOTHELIAL-LIKE CELLS, AND USE THEREFOR**

(30) Priority: 20.05.2021 JP 2021085121
(71) Applicant: Public University Corporation Nagoya City University, Mizuho-ku Nagoya-shi Aichi 467-8601 (JP)
(72) Inventor: MATSUNAGA Tamihide, Nagoya-shi, Aichi 467-8603 (JP); HASHITA Tadahiro, Nagoya-shi, Aichi 467-8603 (JP); AOKI Hiromasa, Nagoya-shi, Aichi 467-8603 (JP)
(74) Representative: Kuhnen & Wacker Patent- und Rechtsanwaltsbüro PartG mbB
(86) International application number: PCT/JP2022/020827
(87) International publication number: WO 2022/244841

(57) **Abstract**

Provided is a technology allowing for production of brain microvascular endothelial-like cells more similar to brain microvascular endothelial cells *in vivo.* This method of producing brain microvascular endothelial-like cells includes a culturing step of culturing vascular endothelial progenitor cells by using a Laminin511 fragment, Fibronectin, and Collagen type IV.

## Description

### [Technical Field]

The present disclosure relates to a method of producing brain microvascular endothelial-like cells. The present application is based on Japanese Patent Application No. 2021-085121, filed on May 20, 2021, the contents of which are herein incorporated by reference.

### [Background Art]

Brain microvascular endothelial cells (BMECs), one type of cells constituting the blood-brain barrier (BBB), inhibit non-specific entry of substances into the brain parenchyma by strong intercellular adhesion and expression of efflux transporters. In drug discovery, this strong barrier function inhibits transfer of a drug candidate to the brain parenchyma (nerve) side, so that drug development may be discontinued. This necessitates a screening model capable of evaluating pharmacokinetics in the human BBB. PLT 1 demonstrates creation of cells similar to BMECs by using human induced pluripotent stem cells (iPS cells), and expression of tight junction proteins and efflux transporters in such cells.

### [Citation List]

### [Patent Literature]

PTL 1: WO 2011/159572 A

### [Summary of Invention]

### [Technical Problem]

Brain microvascular endothelial-like cells obtained by inducing differentiation of human iPS cells while using the method described in PLT 1 have extremely weak characteristics as vascular endothelial cells (ECs) and further have characteristics as epithelial cells. Therefore, it is suggested that the brain microvascular endothelial-like cells have characteristics different from those of human BMECs *in vivo.* Regarding this point, the present inventors have considered that since factors, such as vascular endothelial growth factor (hereinafter, also referred to as "VEGF"), which are essential for differentiation into vascular cells, are not added in the conventional method, differentiation into BMECs is induced without undergoing through vascular endothelial progenitor cells (EPCs), namely progenitors of BMECs. Then, the present inventors have found that in order to produce cells further mimicking (more similarity to) BMECs *in vivo*, it is effective to induce differentiation into BMECs via EPCs. The present inventors have further conducted intensive research and as a result, have invented a technology allowing for production of brain microvascular endothelial-like cells more similar to BMECs *in vivo.*

### [Solution to Problem]

The present invention can be implemented in the following forms.

(1) An aspect of the present invention provides a method of producing brain microvascular endothelial-like cells. The method of producing brain microvascular endothelial-like cells according to this aspect includes a culturing step of culturing vascular endothelial progenitor cells by using a Laminin511 fragment, Fibronectin, and Collagen type IV. The method of producing brain microvascular endothelial-like cells according to this aspect makes it possible to produce brain microvascular endothelial-like cells more similar to brain microvascular endothelial cells *in vivo.*
(2) In the method of producing brain microvascular endothelial-like cells according to the above aspect, the culturing step may include culturing using a medium containing B27 (registered trademark) supplement, A 83-01, and Fibroblast Growth Factor-2 (FGF2). The method of producing brain microvascular endothelial-like cells according to this aspect makes it possible to further improve the barrier function of the brain microvascular endothelial-like cells.
(3) In the method of producing brain microvascular endothelial-like cells according to the above aspect, the vascular endothelial progenitor cells may be cells differentiated from pluripotent stem cells.
(4) In the method of producing brain microvascular endothelial-like cells according to the above aspect, the vascular endothelial progenitor cells may be cells differentiated from human induced pluripotent stem cells. The method of producing brain microvascular endothelial-like cells according to this aspect makes it possible to facilitate production of brain microvascular endothelial-like cells mimicking human brain microvascular endothelial cells *in vivo.*
(5) In the method of producing brain microvascular endothelial-like cells according to the above aspect, the vascular endothelial progenitor cells may be cells differentiated using vascular endothelial growth factor. The method of producing brain microvascular endothelial-like cells according to this aspect makes it possible to produce brain microvascular endothelial-like cells much more similar to brain microvascular endothelial cells *in vivo* because the vascular endothelial progenitor cells are cells differentiated, using vascular endothelial growth factor, from pluripotent stem cells.
(6) In the method of producing brain microvascular endothelial-like cells according to the above aspect, the culturing step may include co-culturing the vascular endothelial progenitor cells with brain pericytes. The method of producing brain microvascular endothelial-like cells according to this aspect makes it possible to produce brain microvascular endothelial-like cells much more similar to brain microvascular endothelial cells *in vivo* because the vascular endothelial progenitor cells are co-cultured with brain pericytes.
(7) In the method of producing brain microvascular endothelial-like cells according to the above aspect, the brain pericytes may be cells differentiated, using an A 83-01-containing medium, from pluripotent stem cells. The method of producing brain microvascular endothelial-like cells according to this aspect makes it possible to further mimic the brain pericytes *in vivo* by using the A-83-01-containing medium.
(8) Another aspect of the present invention provides a method for evaluating permeability of a test substance across a blood-brain barrier by using a cell layer of brain microvascular endothelial-like cells obtained by the method of producing brain microvascular endothelial-like cells according to the above aspect. The method for evaluating blood-brain barrier permeability according to this aspect makes it possible to increase the accuracy of evaluating the permeability of a test substance across the blood-brain barrier because a cell layer of brain microvascular endothelial-like cells mimicking brain microvascular endothelial cells *in vivo.*
(9) The method for evaluating permeability of a test substance across a blood-brain barrier according to the above aspect may comprise the following steps (i) to (iii):
   (i) preparing the cell layer;
   (ii) bringing the test substance into contact with the cell layer; and
   (iii) evaluating permeability of the test substance across the blood-brain barrier by quantifying the test substance having permeated the cell layer.

The method for evaluating blood-brain barrier permeability according to this aspect makes it possible to further increase the accuracy of evaluating the permeability of a test substance across the blood-brain barrier.

(10) Another aspect of the present invention provides a method for evaluating an effect of a test substance on a blood-brain barrier function by using a cell layer of brain microvascular endothelial-like cells obtained by the method of producing brain microvascular endothelial-like cells according to the above aspect. The method for evaluating an effect of a test substance on a blood-brain barrier function according to this aspect makes it possible to increase the accuracy of evaluating an effect of a test substance on a blood-brain barrier function because a cell layer of brain microvascular endothelial-like cells mimicking brain microvascular endothelial cells *in vivo* is used.

(11) Another aspect of the present invention provides brain microvascular endothelial-like cells produced by the method of producing brain microvascular endothelial-like cells according to the above aspect.

(12) In the brain microvascular endothelial-like cells of the above aspect, the expression level of PECAM1 may be higher than that in primary cultured brain microvascular endothelial cells and immortalized brain microvascular endothelial cells, and the TEER value may be 50 S2 × cm² or higher.

(13) Another aspect of the present invention provides a method of inducing differentiation of brain microvascular endothelial-like cells. The method of inducing differentiation of brain microvascular endothelial-like cells according to this aspect includes a culturing step of culturing vascular endothelial progenitor cells by using Laminin511 fragment, Fibronectin, and Collagen type IV. The method of inducing differentiation of brain microvascular endothelial-like cells according to this aspect makes it possible to produce brain microvascular endothelial-like cells mimicking brain microvascular endothelial cells *in vivo.*

### [Brief Description of Drawings]

Fig. 1 is a scheme illustrating a protocol for differentiating human iPS cells into iEPCs.
Fig. 2 is images illustrating the results of checking the properties of iEPCs on day 14 of differentiation.
Fig. 3 is a graph illustrating comparison of electric resistance values between unfrozen iEPCs and frozen iEPCs.
Fig. 4 is a graph illustrating the results of examining conditions for inducing differentiation into iBMELCs (type of medium component).
Fig. 5 is a graph illustrating the results of examining conditions for inducing differentiation into iBMELCs (concentration of medium component).
Fig. 6 is a graph illustrating the results of examining conditions for inducing differentiation into iBMELCs (concentration of A 83-01).
Fig. 7 is a graph illustrating the results of examining conditions for inducing differentiation into iBMELCs (kind of coating component).
Fig. 8 is a graph illustrating the results of examining conditions for inducing differentiation into iBMELCs (kind of coating component).
Fig. 9 is a graph illustrating the results of examining conditions for inducing differentiation into iBMELCs (concentration of LN511F.
Fig. 10 is a graph illustrating the results of examining conditions for inducing differentiation into iBMELCs (cell count).
Fig. 11 is a graph indicating a change in TEER value over time under each differentiation-inducing condition.
Fig. 12 is graphs illustrating the results of a test for permeability of FD4 or LY under each differentiation-inducing condition.
Fig. 13 is images and graphs illustrating the results of protein expression analysis.
Fig. 14 is graphs illustrating the results of gene expression analysis.
Fig. 15 is a graph illustrating comparison of electrical resistance values in iEPCs and immortalized cells.
Fig. 16 is a graph illustrating the results of analyzing the function of P-glycoprotein (P-gp).
Fig. 17 is a scheme for explaining an outline of co-culture of iEPCs and iBPCs.
Fig. 18 is a graph illustrating a change in TEER value over time when iEPCs and iBPCs were co-cultured.
Fig. 19 is images illustrating the results of immunostaining each brain pericyte marker protein.
Fig. 20 is graphs illustrating the results of analyzing the expression level of each brain pericyte marker gene.
Fig. 21 is images illustrating the results of immunostaining each brain pericyte marker protein.
Fig. 22 is graphs illustrating the results of analyzing expression of each brain pericyte marker protein.
Fig. 23 is microscopic images showing the cell migration ability and proliferation potential of iBPCs.
Fig. 24 is a graph illustrating the cell migration ability and proliferation potential of iBPCs.

### [Description of Embodiments]

An embodiment of the present disclosure provides a method of producing brain microvascular endothelial-like cells (hereinafter, also referred to as "BMELCs"). This production method includes a culturing step of culturing vascular endothelial progenitor cells (hereinafter, also referred to as "EPCs") by using a Laminin511 fragment, Fibronectin, and Collagen type IV (type IV collagen).

The EPCs used in the method of the present disclosure may be cells induced to differentiate from pluripotent stem cells, may be cells derived from a living body, or may be immortalized cells. As used herein, the wording "inducing differentiation" refers to making cells differentiate along a specific cell lineage(s). As used herein, the "pluripotent stem cell" refers to a cell having both the ability to differentiate into all cells constituting a living body (differentiation pluripotency) and the ability to produce daughter cells having the same differentiation potential as that of the self through replication (self-renewability). The differentiation pluripotency may be evaluated by transplanting cells to be evaluated into a nude mouse and by testing the presence or absence of formation of teratoma including respective cells from three germ layers (ectoderm, mesoderm, and endoderm).

Examples of the pluripotent stem cells include embryonic stem cells (ES cells), embryonic germ cells (EG cells), or induced pluripotent stem cells (iPS cells). The pluripotent stem cells are not limited thereto as long as having both differentiation pluripotency and self-renewability. As the pluripotent stem cells, iPS cells are preferably used from the viewpoint of availability and ethics. The pluripotent stem cells used are preferably mammalian cells. Examples of the mammal include, but are not particularly limited to, primates (e.g., humans, chimpanzees) or rodents (e.g., mice, rats). Human cells are more preferably used as the pluripotent stem cells. Thus, human iPS cells are particularly preferably used as the pluripotent stem cells. EPCs differentiated from human iPS cells may be used.This makes it possible to facilitate production of BMELCs more similar to human BMECs *in vivo.*

ES cells can be established, for example, by culturing an early embryo before implantation, an inner cell mass constituting the early embryo, or a single blastomere (Manipulating the Mouse Embryo A Laboratory Manual, Second Edition, Cold Spring Harbor Laboratory Press (1994); Thomson, J. A. et al., Science, 282, 1145-1147 (1998)). As the early embryo, an early embryo produced by nuclear transfer of a somatic cell nucleus may be used (Wilmut et al. (Nature, 385, 810 (1997)), Cibelli et al. (Science, 280, 1256 (1998)), Akira Iriya et al. ("Protein, Nucleic Acid, Enzyme", 44, 892 (1999)), Baguisi et al. (Nature Biotechnology, 17, 456 (1999)), Wakayama et al. (Nature, 394, 369 (1998); Nature Genetics, 22, 127 (1999); Proc. Natl. Acad. Sci. USA, 96, 14984 (1999)), Rideout III et al. (Nature Genetics, 24, 109 (2000)), Tachibana et al. (Human Embryonic Stem Cells Derived by Somatic Cell Nuclear Transfer, Cell (2013) in press)). Each parthenogenetic embryo may be used as the early embryo (Kim et al. (Science, 315, 482-486 (2007)), Nakajima et al. (Stem Cells, 25, 983-985 (2007)), Kim et al. (Cell Stem Cell, 1, 346-352 (2007)), Revazova et al. (Cloning Stem Cells, 9, 432-449 (2007)), Revazova et al. (Cloning Stem Cells, 10, 11-24 (2008))). Regarding the ES cell production, one can consult, in addition to the above research articles, for instance, Strelchenko N., et al. Reprod Biomed Online. 9: 623-629, 2004; Klimanskaya I., et al. Nature 444: 481-485, 2006; Chung Y., et al. Cell Stem Cell 2: 113-117, 2008; Zhang X., et al. Stem Cells 24: 2669-2676, 2006; and Wassarman, P. M. et al. Methods in Enzymology, Vol. 365, 2003. Note that each fused ES cell obtained by cell fusion of an ES cell and a somatic cell is also herein included in the embryonic stem cells.

Some ES cells are available from stock institutions or are commercially available. For example, human ES cells are available from, for instance, the Institute for Frontier Life and Medical Sciences, Kyoto University (e.g., KhES-1, KhES-2 and KhES-3), or WiCell Research Institute, ESI BIO. In addition, ES cells may be established by culturing primordial germ cells in the presence of LIF, bFGF, and SCF (Matsui et al., Cell, 70, 841 -847 (1992), Shamblott et al., Proc. Natl. Acad. Sci. USA, 95 (23), 13726 -13731 (1998), Turnpenny et al., Stem Cells, 21(5), 598-609, (2003)).

An iPS cell is a cell that can be produced through reprogramming of a somatic cell, for example, by introducing reprograming factors and has pluripotency (differentiation pluripotency) and proliferation potential. iPS cells exhibit characteristics close to those of ES cells. The somatic cell used for producing the iPS cell is not particularly limited, and may be a differentiated somatic cell or an undifferentiated stem cell. Also, the origin of the somatic cell is not particularly limited, and the somatic cell used is preferably a mammalian (e.g., primates such as humans or chimpanzees, rodents such as mice or rats) somatic cell, and particularly preferably a human somatic cell. The iPS cells can be produced by various methods reported so far. In addition, it is naturally presumed that iPS cell production methods to be developed in the future are also applicable.

The most basic technique for the iPS cell production methods is a method in which four factors Oct3/4, Sox2, Klf4, and c-Myc, which are transcription factors, are introduced into a cell by using viruses (Takahashi K, Yamanaka S: Cell 126 (4), 663-676, 2006; Takahashi, K, et al: Cell 131 (5), 861-72, 2007). Human iPS cells have been reportedly established by introduction of four factors Oct4, Sox2, Lin 28, and Nonog (Yu J, et al: Science 318 (5858), 1917-1920, 2007). Also, iPS cells have been reportedly established by introduction of three factors excluding c-Myc (Nakagawa M, et al: Nat. Biotechnol. 26 (1), 101 -106, 2008), two factors of Oct3/4 and Klf4 (Kim J B, et al: Nature 454 (7204), 646 -650, 2008), or only Oct 3/4 (Kim J B, et al: Cell 136 (3), 411-419, 2009). In addition, techniques for introducing each protein, which is an expression product of each gene, into a cell (Zhou H, Wu S, Joo JY, et al: Cell Stem Cell 4, 381 -384, 2009; Kim D, Kim CH, Moon JI, et al: Cell Stem Cell 4, 472 -476, 2009) have also been reported. Meanwhile, it has also been reported that use of a histone methyl transferase G9a inhibitor (BIX-01294) or a histone deacetylase inhibitor (e.g., valproic acid (VPA) or Bay K 8644) makes it possible to improve the production efficiency and to decrease the number of factors to be introduced (Huangfu D, et al: Nat. Biotechnol. 26 (7), 795-797, 2008; Huangfu D, et al: Nat. Biotechnol. 26 (11), 1269 - 1275, 2008; Silva J, et al: PLoS. Biol. 6 (10), e253, 2008). The gene introduction procedure has also been studied, and other than the retrovirus, each technique using a lentivirus (Yu J, et al: Science 318(5858), 1917-1920, 2007), adenovirus (Stadtfeld M, et al: Science 322 (5903), 945-949, 2008), plasmid (Okita K, et al: Science 322 (5903), 949-953, 2008), transposon vector (Woltjen K, Michael IP, Mohseni P, et al: Nature 458, 766-770, 2009; Kaji K, Norrby K, Pac a, et al: Nature 458, 771-775, 2009; Yusa K, Rad R, Takeda J, et al: Nat Methods 6, 363-369, 2009), or episomal vector (Yu J, Hu K, Smuga-Otto K, Tian S, et al: Science 324, 797-801, 2009) for gene introduction has been developed.

It is possible to sort cells having been transformed into iPS cells, that is, reprogrammed cells by using, as indicators, expression of pluripotent stem cell markers (undifferentiated markers) such as Fbxo 15, Nanog, Oct/4, Fgf-4, Esg-1, and Cript. The sorted cells are collected as iPS cells.

Here, iPS cells may also be obtained, for example, from the National University Corporation Kyoto University or the National RIKEN BioResource Center. In addition, iPS cells can also be provided from, for example, REPROCELL Inc.

Pluripotent stem cells such as iPS cells may be differentiated into EPCs by using, for example, the method described in WO 2020/179380 A or the method described in JP 2018-110548 A. For example, the method may include a step of differentiating pluripotent stem cells into EPCs, and a step of purifying the EPCs by using a difference in adhesion ability between the EPCs constituting the cell population obtained in the step and other cells.

In the step of differentiating pluripotent stem cells into EPCs, the pluripotent stem cells are cultured under conditions that induce differentiation into EPCs. For example, two-step differentiation induction described below, that is, the step of differentiating pluripotent stem cells into mesoderm and the step of differentiating the obtained cells into EPCs may be performed so that pluripotent stem cells are differentiated via mesoderm into EPCs.

The step of differentiating pluripotent stem cells into mesoderm is, for example, in accordance with a past report (see, for example, Sriram G. et al., Efficient differentiation of human embryonic stem cells to arterial and venous endothelial cells under feeder- and serum-free conditions. Stem Cell Research & Therapy 2015, 6: 261) as follows. The cells may be cultured in the presence of a GSK-3β inhibitor by using a medium containing the GSK-3β inhibitor, and then cultured in the presence of basic fibroblast growth factor (bFGF) by using a medium containing the bFGF. Note that in general, bFGF is also called fibroblast growth factor 2 (FGF2). In the step of differentiating the obtained cells into EPCs, for example, differentiation into EPCs may be induced using, for instance, bone morphogenetic protein 4 (BMP4), vascular endothelial growth factor (VEGF), and/or bFGF as a differentiation inducing factor(s).

Other culture conditions (e.g., a culture temperature) in the step of differentiating pluripotent stem cells into EPCs may be conditions generally adopted in culture of animal cells. For example, the cells may be cultured under conditions at 37°C and 5% CO₂. As the basal medium, it is possible to use, for example, a basal medium (e.g., a serum-free medium for vascular endothelial cells (Human Endothelial-SFM), Dulbecco's modified Eagle's medium (D-MEM), Iscove's modified Dulbecco's medium (IMDM), MEM α medium, Ham's F12 medium (HamF12), Glasgow basal medium, RPMI 1640 medium, or MCDB 107 medium). Alternatively, two or more kinds of basal medium such as a mixed medium of D-MEM and Ham's F12 medium may be mixed and used. In addition, other optional components such as serum, serum replacement, antibiotics, and/or supplements may be added to the medium.

Differentiation into EPCs can be determined and evaluated, for example, by using expression of a vascular endothelial progenitor cell marker(s) as an indicator. Examples of the vascular endothelial progenitor cell marker include PECAM1 (CD31), CD34, CDH5 (VE-Cadherin), or FLK1 (VEGFR-2). Among them, CD34 is specific for EPCs and is a particularly useful vascular endothelial progenitor cell marker.

In the step of purifying the EPCs, the ratio of the EPCs constituting the cell population is increased. It is possible to purify the EPCs by preferentially or selectively detaching and removing other cells having a low adhesion ability by utilizing the fact that the EPCs exhibit a higher adhesion ability than the other cells.

The purified cells (high-purity EPCs) may be subjected to culture for maintenance and expansion, or may be stored until use. The preservation method may be in accordance with a conventional method, and for example, cryopreservation may be performed, for instance, at -80°C while using, for example, TC protector (DS Pharma Biomedical Co., Ltd.), Cell Banker (ZENOAQ), Stem Cell Banker (ZENOAQ), or Cell Reservoir One (NACALAI TESQUE, INC.). Cryopreservation can be used to stock EPCs.

The method of producing BMELCs according to the present disclosure includes a culturing step of culturing EPCs by using a Laminin511 fragment, Fibronectin, and Collagen type IV (type IV Collagen) (hereinafter, also called "step A") as described above. In step A, three components of Laminin511 fragment (hereinafter, also referred to as "LN511F"), Fibronectin (hereinafter, also referred to as "FBN"), and Collagen type IV (hereinafter, also referred to as "COL4") are preferably used as a coating agent. Here, the coating agent refers to a coating agent containing a basement membrane component and for forming a coating layer on cultureware for culturing cells.

The three components of LN511F, FBN, and COL4 may be diluted with, for instance, a culture medium, poured into cultureware (e.g., a culture dish), and allowed to stand, resulting in formation of a coating layer containing these three components. Hereinafter, the coating layer formation is also called "coating". In general, cells may be cultured on a coating layer formed using a coating agent, for example, in order to improve the survival and proliferation of cells, promote differentiation induction, and select cells. In the method of producing BMELCs according to the present disclosure, the barrier function of BMELCs can be advantageously improved by using LN511F in addition to FBN and COL4.

Laminin is a heterotrimeric molecule consisting of three subunit chains of an α chain, a β chain, and a γ chain. It is known that the α chain has five types of α1 to α5, the β chain has three types of β1 to β3, and the γ chain has three types of γ1 to γ3. The following has been known as Reference Sequence RNA of Laminin, including NM_000426, NM_001079823 (Subunit alpha2), NM_002291 (Subunit beta1), NM_002292 (Subunit beta2), NM_000228 (Subunit beta3), NM_001318046, NM_001318047, NM_001318048, NM_007356 (Subunit beta4), NM_002293 (Subunit gamma 1), NM_005562, or NM_018891 (Subunit gamma 2).

Laminin511 is a laminin molecule composed of α5, β1, and γ1 subunit chains. As used herein, the "Laminin511 fragment (LN511F)" means a fragment (E8 fragment) corresponding to an Integrin binding site in Laminin511. In addition, the LN511F herein also includes any protein having high identity to LN511F. As used herein, the "protein having high identity to LN511F" refers to a protein in which the amino acid sequence constituting the protein contains 80% or more identical amino acid sequence to the amino acid sequence of LN511F. That is, the "protein having high identity to LN511F" also includes a protein having an amino acid sequence longer than that of LN51 1F. From the viewpoint of enhancing the barrier function of BMELCs, the homology of a protein having high identity to LN511F is preferably 85% or more, more preferably 90% or more, and still more preferably 95% or more.

The LN511F content is not particularly limited, but is preferably 1 µg/mL or larger, more preferably 5 µg/mL or larger, and still more preferably 10 µg/mL or larger in the coating agent used in step A from the viewpoint of enhancing the barrier function of BMELCs. From the viewpoint of cost performance, the LN511F content in the coating agent used in step A is preferably 200 µg/mL or less and more preferably 100 µg/mL or less.

The FBN content is not particularly limited, but is preferably 10 µg/mL or larger and more preferably 100 µg/mL or larger in the coating agent used in step A from the viewpoint of enhancing the barrier function of BMELCs. From the viewpoint of cost performance, the FBN content in the coating agent used in step A is preferably 500 µg/mL or less and more preferably 200 µg/mL or less.

The COL4 content is not particularly limited, but is preferably 100 µg/mL or larger and more preferably 400 µg/mL or larger in the coating agent used in step A from the viewpoint of enhancing the barrier function of BMELCs. From the viewpoint of cost performance, the COL4 content in the coating agent used in step A is preferably 2000 µg/mL or less and more preferably 1000 µg/mL or less.

The total content of three components LN511F, FBN, and COL4 is not particularly limited, but is preferably 100 µg/mL or larger, more preferably 300 µg/mL or larger, and still more preferably 500 µg/mL or larger in the coating agent used in step A from the viewpoint of enhancing the barrier function of BMELCs. From the viewpoint of cost performance, the total content of three components LN511F, FBN, and COL4 in the coating agent used in step A is preferably 2000 µg/mL or less and more preferably 1000 µg/mL or less. In addition, the content ratio between LN511F and FBN in the coating agent used in step A is not particularly limited, but is preferably 1:1 to 1:100 and more preferably 1:2 to 1:20 from the viewpoint of enhancing the barrier function of BMELCs. Further, the content ratio between LN511F and COL4 in the coating agent used in step A is not particularly limited, but is preferably 1:4 to 1:400 and more preferably 1:8 to 1:80 from the viewpoint of enhancing the barrier function of BMELCs.

The total content of the three components LN511F, FBN, and COL4 is preferably 20 ng or larger in total, and more preferably 100 ng or larger in total per the area of, for instance, a culture dish, that is, per cm² of the coated area on the coating layer. The total content of the three components LN511F, FBN, and COL4 is preferably 200 µg or less in total and more preferably 40 µg or less in total per cm² of the coated area on the coating layer. In addition, note that examples of the culture dish that can be used include a petri dish, a cell culture plate having a plurality of wells, or cultureware in any form. Also, the coating agent and the coating layer may contain other optional components as long as the effects of the invention are not impaired. Examples of the optional component include, but are not particularly limited to, Vitronectin, various Laminins, Agrin, gelatin, or poly-L-lysine.

The above step A can be performed using various media. Examples of the medium include a basal medium (e.g., a serum-free medium for vascular endothelial cells (Human Endothelial-SFM), Dulbecco's modified Eagle's medium (D-MEM), Iscove's modified Dulbecco's medium (IMDM), MEM α medium, Ham's F12 medium (HamF12), Glasgow basal medium, RPMI 1640 medium, or MCDB 107 medium). Note that as the medium, two or more different basal media may be mixed and used.

In the culturing step (step A), a medium containing B27 (registered trademark) supplement (hereinafter, also referred to as "B27s"), A 83-01, and Fibroblast Growth Factor-2 (FGF2) may be preferably used for culture A culture medium containing B27s, A 83-01, and FGF2 may be used for culture to further improve the barrier function of BMELCs.

B27s is a cell culture supplement and is available from Thermo Fisher Scientific. The B27s content is preferably 0.5% or larger, more preferably 2.5% or larger, and still more preferably 7% or larger in the culture medium used in step A from the viewpoint of enhancing the barrier function of BMELCs. From the viewpoint of cost performance, the B27s content in the culture medium used in step A is preferably 15% or less and more preferably 10% or less.

A 83-01 is a kind of TGF-β inhibitor. More specifically, A 83-01 is a selective inhibitor of TGF-β type I/activin receptor-like kinase (ALK5), type I activin/nodal receptor-like kinase (ALK4), and type I nodal receptor-like kinase (ALK7). The A 83-01 content is preferably 10 nM or larger and more preferably 0.1 µM or larger in the culture medium used in step A from the viewpoint of enhancing the barrier function of BMELCs. From the viewpoint of cost performance, the A 83-01 content in the culture medium used in step A is preferably 100 µM or less and more preferably 10 µM or less.

FGF2 means fibroblast growth factor 2, also called basic fibroblast growth factor (bFGF). The FGF2 may be, for example, human FGF2 (e.g., human recombinant FGF2). The FGF2 content is preferably 1 ng/mL or larger and more preferably 10 ng/mL or larger in the culture medium used in step A from the viewpoint of enhancing the barrier function of BMELCs. From the viewpoint of cost performance, the FGF2 content in the culture medium used in step A is preferably 10 µg/mL or less and more preferably 1 µg/mL or less.

The culture medium used in step A may contain other optional components as long as the effects of the invention are not impaired. Examples of the optional component include, but are not particularly limited to, serum (e.g., fetal bovine serum, human serum, sheep serum), serum replacement (e.g., Knockout serum replacement (KSR)), antibiotics (e.g., penicillin, streptomycin), supplements (e.g., ITS-G supplement), L-glutamine, L-ascorbic acid phosphate magnesium salt n-hydrate, non-essential amino acids (NEAA), 2-mercaptoethanol, or Chemically Defined Lipid Concentrate (Gibco).

The culture period in step A is not particularly limited, but may be, for example, from 1 day to 10 days, and preferably from 2 days to 5 days. This culture period makes it possible to suppress deterioration of the efficiency of inducing differentiation into BMELCs, and also enables the barrier function of BMELCs to be enhanced. In addition, the cells may be subcultured in the middle of inducing differentiation. For example, when the cells become confluent or sub-confluent, part of the cells may be collected and transferred to another cultureware. The culture may then be continued. At the time of cell recovery associated with, for instance, medium change or subculture, the cells may be treated in advance with a ROCK (Rho-associated coiled-coil forming kinase/Rho-binding kinase) inhibitor such as Y-27632 in order to suppress cell death.

Other culture conditions (e.g., culture temperature) may be conditions generally employed in the culture of animal cells. For example, the cells may be cultured under conditions at 37°C and 5% CO₂. In addition, the method is not limited to the method of two-dimensionally culturing cells using, for instance, a culture dish, and three-dimensional culture may be performed using, for instance, a three-dimensional culture plate including a coating layer coated with the coating agent.

The method of producing BMELCs according to the present disclosure may include a step of inducing differentiation of pluripotent stem cells into vascular endothelial progenitor cells (hereinafter, also referred to as "step B") before the culturing step (step A). Meanwhile, step B may be a step of inducing differentiation of pluripotent stem cells into vascular endothelial progenitor cells by using vascular endothelial growth factor.

In the method of producing BMELCs according to the present disclosure, step A, that is, the culturing step of culturing EPCs may include co-culturing EPCs with brain pericytes (hereinafter, also referred to as "BPCs"). In general, brain pericytes, also called pericytes, constitute the blood-brain barrier together with brain microvascular endothelial cells.

As used herein, "co-culturing EPCs and BPCs" means a state in which EPCs and BPCs are cultured together. In the co-culture, the EPCs and the BPCs may be present in the same medium (culture medium) or in media (culture media) different from each other. In the co-culture, the EPCs and the BPCs may be in a state where cells are in contact with each other or in a non-contact state, but are preferably in a non-contact state. The non-contact state corresponds to, for example, a state in which EPCs and BPCs exist separately on the front surface side and the back surface side, respectively, via, for instance, a microporous support membrane, or a state in which EPCs and BPCs exist separately at a distance in the culture medium via, for instance, a support. The support is not particularly limited, but may be composed of, for example, a supporting membrane and a supporting tool. The support is a member that supports EPCs and BPCs and fixes the supporting membrane to cultureware. The support may be specifically a top-hat-shaped cell culture insert. The supporting membrane is preferably microporous, and the size of each pore is preferably a size that the cell cannot pass through and a size that the cell protrusion and the culture medium, for example, can pass through. The supporting membrane may be formed of any material that does not interfere with the maintenance, survival, and differentiation induction of cells.

The BPCs used in the co-culture with EPCs may be cells differentiated from pluripotent stem cells, may be cells derived from a living body, or may be immortalized cells. As the BPCs, it is preferable to use cells differentiated from pluripotent stem cells from the viewpoint of availability, and it is more preferable to use iPS cells from the viewpoint of ethics.

The method of producing BMELCs according to the present disclosure may include a step of inducing differentiation of pluripotent stem cells into brain pericytes (hereinafter, also referred to as "step C") before the culturing step (step A).

In step C, pluripotent stem cells are cultured under conditions that induce differentiation into BPCs. For example, two-step differentiation induction described below may be performed so that pluripotent stem cells differentiate via mesoderm into BPCs. That is, the step of differentiating pluripotent stem cells into mesoderm and the step of differentiating the obtained cells into BPCs may be implemented.

The step of differentiating pluripotent stem cells into mesoderm may be performed, for example, by the above-described method. In the step of differentiating the obtained cells into BPCs, for example, differentiation into BPCs may be induced using, for instance, platelet-derived growth factor (PDGF-BB) as a differentiation inducing factor.

Other culture conditions (e.g., culture temperature) in step C may be conditions generally employed in the culture of animal cells. The culture conditions may be set to, for example, 37°C and 5% CO₂. As the basal medium, it is possible to use, for example, a basal medium (e.g., a serum-free medium for vascular endothelial cells (Human Endothelial-SFM), Dulbecco's modified Eagle's medium (D-MEM), Iscove's modified Dulbecco's medium (IMDM), MEM α medium, Ham's F12 medium (HamF12), Glasgow basal medium, RPMI 1640 medium, MCDB 107 medium, EGM-2 basal medium). Alternatively, two or more kinds of basal medium such as a mixed medium of D-MEM and Ham's F12 medium may be mixed and used. In addition, other optional components such as serum, serum replacement, antibiotics, and/or supplements may be added to the medium. In step C, for example, a coating agent such as Vitronectin-N (VTN-N) may be used.

Differentiation into BPCs can be determined and evaluated, for example, by using expression of a pericyte marker(s) as an indicator. Examples of the pericyte marker include PDGFR-β, NG2, or α-SMA. BPCs are preferably negative for expression of α-SMA from the viewpoint of mimicking cells *in vivo.* Therefore, negative expression of α-SMA can be used as a useful indicator for distinguishing between the brain pericytes and peripheral pericytes.

The BPCs used in step A may be cells differentiated from pluripotent stem cells by using a culture medium containing A 83-01. Therefore, step C may include a step of inducing differentiation of pluripotent stem cells into brain pericytes by using a medium containing A 83-01. Use of a medium containing A 83-01 allows the cells to be much more similar to BPCs. The A 83-01 content is preferably 10 nM or larger and more preferably 0.1 µM or larger in the culture medium used in step C from the viewpoint of enhancing the cell migration ability and cell proliferation potential of BPCs. From the viewpoint of cost performance, the A 83-01 content in the culture medium used in step C is preferably 100 µM or less and more preferably 10 µM or less.

When the EPCs are co-cultured with the BPCs in step A, step C and step A may be continuously performed. That is, the cultureware (e.g., a cell culture insert) used in step C may be used as it is, and step A may be continuously performed while appropriately changing the culture medium. More specifically, first, step C may include seeding pluripotent stem cells on the outer side (bottom back surface) of a cell culture insert to induce differentiation of the pluripotent stem cells into BPCs. During the differentiation induction, it is preferable to add A 83-01 to the culture medium. Thereafter, EPCs may be seeded on the inner side (bottom top surface) of the cell culture insert, and the EPCs may be co-cultured with BPCs while using LN511F, FBN, and COL4.

The method of the present disclosure makes it possible to produce cells more similar to BMECs *in vivo,* that is, BMELCs that mimic BMECs *in vivo.*

In addition, the method of co-culturing EPCs with BPCs in the culturing step of inducing differentiation of EPCs into BMELCs makes it possible to produce BMELCs much more similar to BMECs *in vivo.* Further, the cells can be more similar to BPCs *in vivo* in an embodiment of using a medium containing A 83-01 when BPCs to be co-cultured with EPCs are induced to differentiate from pluripotent stem cells. This can produce BMELCs much more similar to BMECs *in vivo.*

Here, there is no report on expansion culture of brain microvascular endothelial-like cells obtained by inducing differentiation of iPS cells by conventionally reported methods. Thus, iPS cells should be differentiated every time. For this reason, the conventional methods have a big problem in the cell supply aspect.

By contrast, in the method of producing BMELCs according to the present disclosure, EPCs are induced to differentiate into BMELCs. Here, since EPCs can be expanded and cryopreserved, they are excellent in supply performance. Thus, the method of inducing differentiation of EPCs into BMELCs according to the present disclosure can facilitate production of BMELCs because frozen cells can be used as a differentiation source. In addition, the method of inducing differentiation of EPCs into BMELCs according to the present disclosure can suppress variations in performance due to differences in lots of produced cells.

A monolayer (cell layer) of BMELCs is formed by continuously culturing BMELCs produced by inducing differentiation including the step A under culture conditions suitable for maintenance and expansion of BMELCs. The method of the present disclosure results in a cell layer having an excellent barrier function. The barrier function of the cell layer obtained by the method of the present disclosure can be characterized by strong tight junctions and maintenance of the tight junctions for a long period of time.

The barrier function of the cell layer obtained by the method of the present disclosure can also be further characterized by having a function of important or characteristic drug transporter (e.g., BCRP, P-gp, GLUT1) in the BMECs. In addition, the function of BMELCs may be determined or evaluated using, as an indicator, expression of a tight junction marker (e.g., Claudin 5, Occludin, ZO-1) important for or characteristic of BMECs.

At a later stage of inducing differentiation, a cell layer of BMELCs can be formed on a semi-permeable membrane by culturing the cells on the semi-permeable membrane (porous membrane). This embodiment is particularly effective when the cell layer of BMELCs obtained by the production method of the present disclosure is used for various assays. For example, cultureware (e.g., Transwell (registered trademark), supplied by Corning Inc.) provided with a culture insert (having a culture surface composed of a semi-permeable membrane) can be used to culture cells in the insert to form a cell layer.

Another aspect of the present disclosure relates to use of BMELCs produced by inducing differentiation by the method described above. As described above, according to the present disclosure, a cell layer composed of BMELCs can be obtained, and the cell layer can be used for a BBB model. For example, the present disclosure can be applied to, for instance, BBB pharmacokinetic studies, pathological analysis, and differentiation studies. More specifically, the present disclosure may be used, for instance, for a novel BBB pharmacokinetic evaluation model as the BBB pharmacokinetic study. As the pathological analysis, for example, it can be used for a pathology-reproducing model using disease iPS cells for a disease involved in a BBB failure. As the differentiation study, it can be used for, for instance, applications to BBB development/differentiation studies at the time of embryonic development and BBB inflammation models. For example, an assay using a cell layer composed of BMELCs prepared by the method of the present disclosure can be used for evaluation of intracerebral transfer (blood-brain barrier permeability) of a test substance (e.g., a drug product or a drug candidate). Besides, the evaluation accuracy can be improved. As a result, the permeability of a test substance across the BBB in the human living body can be easily predicted *in vitro.*

According to another aspect of the present disclosure, the cell layer of BMELCs obtained by the above production method may be used to provide a method for evaluating test substance BBB permeability (hereinafter, also referred to as "BBB permeability evaluation method of the present disclosure"). The BBB permeability evaluation method of the present disclosure includes the following steps (i) to (iii):
(i) preparing a cell layer of BMELCs obtained by the production method of the present disclosure;
(ii) bringing a test substance into contact with the cell layer; and
(iii) evaluating permeability of the test substance across the blood-brain barrier by quantifying the test substance having permeated the cell layer.

In step (i), a cell layer of BMELCs obtained by the production method of the present disclosure is prepared. In addition to the cell layer of BMELCs, other cells (e.g., pericytes, astrocytes) may be used in combination. For example, cultureware having a culture insert may be used to form a cell layer of BMELCs in the culture insert (a cell layer is formed on a bottom upper surface of the culture insert); pericytes may be cultured in a state of being adhered to the bottom back surface of the culture insert (pericytes-adhered co-culture system), pericytes may be cultured in a section between the culture insert and each well (pericytes-non-adhered co-culture system), pericytes may be cultured in a state of being adhered to the bottom back surface of the culture insert and astrocytes may be cultured in a section between the culture insert and each well (pericytes-adhered/astrocytes-non-adhered co-culture system), or astrocytes may be cultured in a state of being adhered to the bottom back surface of the culture insert (astrocytes-adhered co-culture system).

The "contact" in step (ii) is typically performed by adding a test substance to a culture medium. The timing of addition of the test substance is not particularly limited. Thus, after the culture in the test substance-free culture medium is started, the test substance may be added at a certain time point, or the culture may be started in a culture medium already containing the test substance.

Typically, a pharmaceutical product or a pharmaceutical candidate substance is used as the test substance. However, the test substance is not particularly limited, and organic compounds or inorganic compounds having various molecular sizes may each be used as the test substance. Examples of the organic compound include nucleic acid, peptide, protein, lipid (simple lipid, complex lipid (e.g., phosphoglyceride, sphingolipid, glycosylglyceride, cerebroside)), prostaglandin, isoprenoid, terpene, steroid, polyphenol, catechin, or vitamin (e.g., B1, B2, B3, B5, B6, B7, B9, B12, C, A, D, E). It is also possible to use, as the test substance, for example, plant extract, cell extract, or culture supernatant. Two or more kinds of test substances may be simultaneously added to investigate, for instance, interaction and/or synergy between the test substances. The test substance may be derived from a natural product or may be synthesized. In the latter case, for example, an efficient assay system may be constructed using a combinatorial synthesis technique.

The period during which the test substance is brought into contact may be optionally set. The contact period is, for example, from 10 minutes to 3 days and preferably from 1 hour to 1 day.

In step (iii), the test substance having permeated the cell layer is quantified. For example, in a case where cultureware having a culture insert such as Transwell (registered trademark) is used, the test substance having permeated through the culture insert, that is, the test substance having moved into the upper container (culture insert) or the lower container (well) through the cell layer is quantified by an assay protocol corresponding to the test substance. Examples of the assay protocol include an assay protocol such as mass spectrometry, liquid chromatography, or immunological technique (e.g., fluorescent immunoassay (FIA), enzyme immunoassay (EIA)). The membrane permeability of the test substance is evaluated based on the quantification results (the amount of test substance having permeated the cell layer) and the amount of test substance used (typically, the amount added to the culture medium). In addition to the membrane permeability, for instance, absorption by the cell layer (absorbability), the effect on the cell layer (e.g., the effect on the barrier function), and/or the effect on the expression or function of a transporter (e.g., BCRP or P-gp) may be evaluated. Generally speaking, the absorbability and the permeability have a front-back relation. Thus, they may be evaluated by the same method as in the case of permeability. The effect on the barrier function may be evaluated, for example, by measuring the TEER value or conducting a permeability test using a non-absorbable marker. In addition, the effect on the expression of a transporter may be evaluated by, for instance, an immunological technique, Western blotting, or flow cytometry, and the effect on the corresponding function may be evaluated, for example, by an activity test using a substrate.

As can be seen from the above description, the effect of the test substance on the BBB function (e.g., improvement, deterioration, and failure of the BBB function) may also be evaluated using the cell layer of BMELCs. Accordingly, as another application of the cell layer of BMELCs produced by the production method of the present disclosure, the present disclosure also provides a method for evaluating the BBB function as a target or subject, that is, a method for evaluating an effect of a test substance on the BBB barrier function. This evaluation method is useful, for example, as a means for searching for a substance that enhances (improves) the barrier function, a substance that protects the barrier function, a substance that modulates the barrier function, and so on. The method may also be used for evaluation of toxicity to the BBB. In this evaluation method, like in the BBB permeability evaluation method, a step of preparing a cell layer and a step of bringing a test substance into contact with the cell layer are performed, and the effect on the barrier function of the cell layer is then evaluated. The procedure for evaluating the effect on the barrier function is as described above.

Another aspect of the present disclosure provides brain microvascular endothelial-like cells produced by the above production method. In addition, another aspect of the present disclosure provides brain microvascular endothelial-like cells having a higher expression level of PECAM1 than that in primary cultured brain microvascular endothelial cells and immortalized brain microvascular endothelial cells, and having a transendothelial electrical resistance (TEER value) of 50 S2 × cm² or higher. Such brain microvascular endothelial-like cells can be obtained, for example, by the production method described above.

The relative expression level of PECAM1 may be quantified using the mRNA expression level or the protein expression level. The mRNA expression level may be quantified using, for example, RT-q PCR. The protein expression level may be quantified, for example, by immunostaining. The TEER value was measured using a Millicell ERS-2 (chopstick type) according to the instructions attached to the device. The TEER value is measured before culture medium change. More specifically, first, the culture dish is allowed to stand at room temperature for about 15 minutes, so that the temperature in the culture medium is returned to room temperature. Next, the TEER value of the cell culture insert not seeded with cells is measured and used as a blank. Then, the TEER value of the cell culture insert seeded with cells is measured. After that, a value obtained by subtracting the TEER value of the blank can be obtained as an actually measured value.

### [Examples]

Hereinafter, the present invention will be described more specifically with reference to Examples. The present invention, however, is not limited to the following Examples.

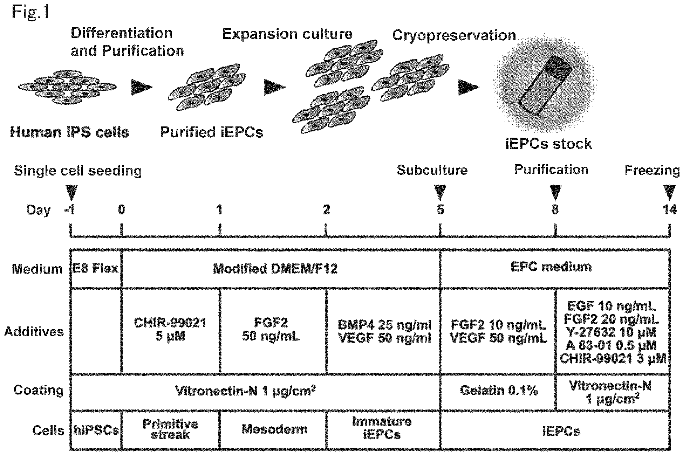

Fig. 1 is an scheme illustrating a protocol of differentiation from human iPS cells (hereinafter, also referred to as "hiPSCs") to human iPS cell-derived vascular endothelial progenitor cells (hereinafter, also referred to as "iEPCs"). In the Examples, as shown in Fig. 1, iEPCs were produced by inducing differentiation of hiPSCs into iEPCs through primitive streak, mesoderm, and immature iEPCs, and then purifying the iEPCs. The iEPCs thus produced were used to induce differentiation into human iPS cell-derived brain microvascular endothelial-like cells (hereinafter, also referred to as "iBMELCs"). Note that in the following description, unless otherwise specified, "%" indicates volume/volume%, and "w/v%" indicates weight/volume%.

### 1. Materials and Methods

### (1) Coating of culture dish

As a coating of a culture dish at the time of inducing differentiation of hiPSCs into iEPCs, Vitronectin-N (VTN-N) coating was prepared by diluting VTN-N (manufactured by Thermo Fisher Scientific) to 1 µg/cm² with D-PBS (-), and allowing the dish to stand at 37°C for 1 to 2 hours. For the Gelatin coating, 0.1% gelatin solution was left to stand at 37°C for 1 hour or at 4°C overnight. As a coating of a culture dish at the time of inducing differentiation of iEPCs into iBMELCs, a two-component coating of Fibronectin and Collagen type IV was prepared by diluting Fibronectin (manufactured by Wako Pure Chemical Industries, Ltd.) to 100 µg/mL and Collagen type IV (manufactured by Nitta Gelatin Inc.) to 400 µg/mL with D-PBS (-), and the dish was allowed to stand at 37°C for 2 to 4 hours. In addition, coating with three components was performed by adding one component to a mixed solution of Fibronectin and Collagen type IV. This third component corresponds to VTN-N (manufactured by Thermo Fisher Scientific), a Laminin221 fragment (LN221F: iMatrix 221 manufactured by Nippi Incorporated), a Laminin411 fragment (LN411F: iMatrix 411 manufactured by Nippi Incorporated), a Laminin511 fragment (LN511F: iMatrix 511 manufactured by Nippi Incorporated), a Laminin-fragment mix (mixture of Laminin221F, Laminin411F, and Laminin511F), Laminin211 (manufactured by Veritas), Laminin411 (manufactured by Veritas), Laminin511 (manufactured by Veritas), Agrin (manufactured by R&D), or a Laminin-mix (mixture of Laminin211, Laminin411, and Laminin511). The concentrations of VTN-N, LN221F, and LN411F were each 10 µg/mL. The concentration of LN511F was from 10 to 200 µg/mL. In addition, coating with 4 components in which LN411F and LN511F were each added at 100 µg/mL was also performed. Collagen type I coating used for culturing a human immortalized cell line was prepared by pouring, on a dish, 0.01% Collagen type I solution (manufactured by Nitta Gelatin Inc.), sucking the solution, and then allowing the dish to stand for drying. For Matrigel GFR coating used in the method according to the report of Lippmann *et al.,* Matrigel GFR (manufactured by Corning Incorporated) was diluted 30 times with a human iPS cell maintenance medium on ice, and allowed to stand at 37°C for 30 minutes or longer.

### (2) Cell culture

First, human iPS cell 610B1 strain (available from Riken Cell Bank) was used and cultured on a VTN-N-coated culture dish using Essential 8 flex medium (manufactured by Thermo Fisher Scientific) as a culture medium. A detachment solution used when the 610B1 strain was subcultured was 0.5 M EDTA (pH 8.0) or TrypLE Select (manufactured by Thermo Fisher Scientific). When TrypLE Select was used for detachment, 10 µM of Y-27632 was added immediately after subculture, and the next day the culture medium was changed.

Human immortalized cell line hCMEC/D3 was obtained from Merck Millipore, and cultured using hCMEC/D3 medium (Endothelial Cell Basal Medium-2 (Lonza) containing 5% fetal bovine serum (from Sigma-Aldrich), 5 µg/mL L-ascorbic acid phosphate magnesium salt n-hydrate, 1% chemically defined lipid concentrate (from Thermo Fisher Scientific), 10 µM HEPES solution (from Sigma-Aldrich), 1% penicillin-streptomycin solution (from Biological Industries), 1.4 µM hydrocortisone (from Wako), and 1 ng/mL FGF2 (manufactured by PeproTech, Inc.)). For detachment at the time of subculture, TrypLE Select was used, and the cells were seeded at 5.0 × 10³ cells/cm² on a Collagen type I-coated culture dish. The culture medium was changed once every two days.

As a comparative example, human iPS cells were induced to differentiate into brain microvascular endothelial-like cells according to the report of Lippmann *et al.* At that time, 610B1 strain was used as human iPS cells and cultured using iPS cell medium (DMEM/F12 (manufactured by Wako Pure Chemical Industries) containing 20% KSR (manufactured by Thermo Fisher Scientific), 1 × MEM nonessential amino acids, 2 mM L-glutamine (manufactured by Wako Pure Chemical Industries), 0.1 mM 2-mercaptoethanol (manufactured by Sigma-Aldrich Corporation)) supplemented with 5 ng/mL FGF2. Note that the cells were cultured on mouse embryonic fibroblasts, the proliferation capability of which was inactivated by mitomycin C treatment. A detachment solution used was D-PBS (-) (manufactured by Takara Bio Inc.) containing 1 mg/mL collagenase IV, 0.25% trypsin, 20% KSR, and 1 mM CaCl₂.

### (3) To induce differentiation into iEPCs

TrypLE Select was used to detach human iPS cells cultured in Essential 8 Flex medium. The cells were seeded at a density of 2 × 10⁴ cells/cm² on a VTN-N-coated culture dish, and cultured for 24 hours in Essential 8 Flex medium containing 10 µM of Y-27632 (manufactured by Focus Biomolecules). Thereafter, the cells were cultured for 1 day in modified DMEM/F12 medium (DMEM/F12 containing 0.1% chemically defined lipid concentrate, 0.1 × insulin-transferrin-selenium (from Thermo Fisher Scientific), 2 mM GlutaMAX (from Sigma-Aldrich), 450 mM 1-thioglycerol (from Sigma-Aldrich), 50 µg/mL of L-ascorbic acid phosphate magnesium salt n-hydrate, and 1 × penicillin-streptomycin solution) supplemented with 5 µM CHIR-99021 (manufactured by Focus Biomolecules). Next, the cells were cultured for 1 day in modified DMEM/F12 medium containing 50 ng/mL of FGF2. Then, the cells were cultured for 3 days in modified DMEM/F12 medium containing 50 ng/mL VEGF (manufactured by Biolegend) and 25 ng/mL BMP4 (manufactured by Prospec). During this time, the medium was changed every day. On day 5, the cells treated with 10 µM Y-27632 for 1 hour were dissociated by TrypLE Select, subcultured at 3.5 × 10⁴ cells/cm² on a new gelatin-coated culture dish, and induced for differentiation in modified Human endothelial (HE)-SFM medium {HE-SFM (from Thermo Fisher Scientific) + 5% knockout serum replacement (from Thermo Fisher Scientific) + 1 × penicillin-streptomycin} supplemented with 50 ng/mL VEGF and 10 ng/mL FGF2. During this time, the medium was changed every day.

### (4) To purify iEPCs

The cells on day 8 of differentiation as treated with 10 µM of Y-27632 for 1 hour were treated with TrypLE Select for 45 to 60 seconds to detach cells (extra cells) other than iEPCs to some extent, and the extra cells were completely detached by hitting (tapping) the culture dish several times in a flicking manner. At this time point, when the cells at the outermost edge of the culture dish were difficult to detach, the cells at the edge of the culture dish were aspirated circumferentially. Thereafter, the cells were washed three times or more with D-PBS (-) and treated again with TrypLE Select. Next, the iEPCs were detached, collected in a tube, centrifuged at 100 × g for 5 minutes, and then reseeded on a new culture dish.

### (5) To culture iEPCs

The purified cells were cultured in modified Human endothelial SFM medium containing 10 ng/mL EGF (manufactured by GenScript), 20 ng/mL FGF2, 10 µM Y-27632, 0.5 µM A 83-01 (manufactured by Adooq Bioscoence), and 3 µM CHIR-99021. The medium was changed on day 1 after seeding (day 9 and day 12 of differentiation). The cells were subcultured on day 11 of differentiation. At the time of subculture, the cells were washed once with D-PBS (-) and then treated with TrypLE Select at 37°C for 5 minutes or longer to detach the cells. Next, the cells were collected into a 15 mL tube or a 50 mL tube with the culture medium and centrifuged at 100 × g for 5 minutes. Then, the cells were resuspended using a culture medium, and reseeded on a VTN-N-coated culture dish at 1 × 10⁴ cells/cm² or 1.5 × 10⁴ cells/cm². It could be judged that the residual ratio of extra cells was large at the time point of day 11 of differentiation so that the cells were not purified enough. In this case, the cells were washed with D-PBS (-), admixed with TrypLE Select, observed under a microscope at normal temperature, and then tapped strongly several times at the stage when the extra cells started detaching. After the detachment solution was aspirated and the cells were washed once with D-PBS (-), TrypLE Select was added again and a routine subculture procedure was applied. The cells were cultured until day 14 of differentiation.

### (6) Cryopreservation and thawing of cells

In the same manner as in the subculture, a cell pellet of iEPCs on day 14 of differentiation was prepared, resuspended in TC Protector (manufactured by DS Pharma Biomedical Co., Ltd.), and then frozen and stored in a deep freezer at -80°C. At the time of thawing, the cells were semi-thawed in a warm bath at 37°C, and 1 mL of the culture medium from 10 mL contained in a 15 mL tube or a 50 mL tube warmed in advance was poured into a semi-thawed cell suspension. The cell suspension was completely thawed, returned to the tube, and then centrifuged at 100 × g for 5 minutes. Thereafter, the cells were resuspended in a culture medium and seeded on a well plate or Transwell (registered trademark). In addition, vascular endothelial progenitor cells prepared from hCMEC/D3 were also cryopreserved and thawed in the same manner.

### (7) To induce differentiation into brain microvascular-like cells

iEPCs were induced to differentiate into iBMELCs under conditions where the coating agent and the additive added to the culture medium each varied. For coating the culture dish, as described above, two components of FBN and COL4, or three components obtained by adding any one component of VTN-N, LN221, LN411F, and LN511F to these two components, or four components obtained by adding LN411F and LN511F to these two components were used. As the third component, Laminin211, Laminin411, Laminin511, Agrin, Laminin-mix, Laminin-fragment mix, or hep ex was also used. Unfrozen or frozen iEPCs (day 14 of differentiation) were seeded, and cultured in HE-SFM medium (+ 1 × penicillin-streptomycin; the same applies to the following) supplemented with 2 to 10% KSR and 20 ng/mL FGF2, HE-SFM medium supplemented with 5% FBS (manufactured by Sigma-Aldrich Corporation) and 20 ng/mL FGF2, HE-SFM medium supplemented with 5% Platelet-poor plasma-derived bovine serum (PDS) (manufactured by Alfa Aesar) and 20 ng/mL FGF2, HE-SFM medium supplemented with 2 to 10% B27 (registered trademark) supplement (manufactured by Thermo Fisher Scientific) and 20 ng/mL FGF2, or HE-SFM medium supplemented with 7.5% B27 supplement, 0.1 to 10 µM A 83-01, and 20 ng/mL FGF2. The cells were induce to differentiate into brain microvascular-like cells. The medium was changed on day 1 after seeding, and the medium was then changed every other day. In addition, in order to examine the cell density when iEPCs were seeded on a culture dish, the iEPCs were seeded on a culture dish coated with 100 µg/mL of Fibronectin, 400 µg/mL of Collagen type IV, and 10 µg/mL of LN511F at 1 × 10⁵ cells/cm², 3 × 10⁵ cells/cm², 5 × 10⁵ cells/cm², 7 × 10⁵ cells/cm², or 9 × 10⁵ cells/cm², and cultured in HE-SFM medium supplemented with 7.5% B27 supplement, 1 µM A 83-01, and 20 ng/mL FGF2. Similarly, human immortalized brain microvascular endothelial cells (hCMEC/D3) were induced to differentiate into brain microvascular-like cells while using differentiation-inducing condition A described later.

### (8) To induce differentiation into iBMELCs by existing method (Comparative Example)

The procedure was performed based on a protocol modified from the report of Lippmann *et al.* Human iPS cells were seeded on a 6-well plate coated with Matrigel-GFR, and cultured in StemSure hPSC medium (manufactured by Wako) supplemented with 35 ng/mL FGF2 for 3 days or 4 days. During this time, the medium was changed daily. On the differentiation start day (60 to 70% confluence), the medium was changed to DMEM/F12-based medium (iPS cell medium), and the cells were cultured for 6 days. During this time, the medium was changed daily. On day 6 of differentiation, the cells were cultured for another 2 days in an HE-SFM-based medium (HE-SFM containing 1% PDS and 1 × Penicillin-streptomycin solution (from Biological Industries USA, Inc.)) containing 10 µM all-trans-retinoic acid (RA) (manufactured by Tocris Bioscience) and 20 ng/mL FGF2. During this time, the medium was changed every day. On day 8 of differentiation, the differentiated cells were washed with D-PBS (-), admixed with an Accutase cell detachment solution, and allowed to stand at 37°C for 20 minutes. Thereafter, the cells recovered using the culture medium were centrifuged at 100 × g for 5 minutes to prepare a cell suspension, seeded on a Fibronectin + Collagen type IV-coated well plate or Transwell (registered trademark) (12 well, pore size: 1 µm), and then cultured in an HE-SFM-based medium supplemented with 10 µM RA and 20 ng/mL FGF2. On day 9 of differentiation, the culture medium was changed to HE-SFM-based medium without RA or FGF2, and the cells were cultured.

### (9) Immunostaining analysis

iEPCs induced to differentiate from hiPSCs by the above method were analyzed by immunostaining in order to check the properties as EPCs. PECAM1 and CD34 antibodies were used in this immunostaining. In addition, protein expression was analyzed by immunostaining on iBMELCs induced to differentiate from frozen iEPCs by the above method using the following differentiation-inducing condition A, and as a Comparative Example, cells cultured using an HE-SFM medium with a coating agent containing 100 µg/mL of Fibronectin and 400 µg/mL of Collagen type IV and supplemented with 7.5% KSR and 20 ng/mL FGF2. PECAM1, CDH5, ZO-1, CLAUDIN5, P-gp, BCRP and GLUT1 antibodies were used in this immunostaining. The cells on the 96-well plate were washed 3 times with 0.1% BSA-containing D-PBS (-), then fixed in 4% paraformaldehyde for 15 minutes, washed again with 0.1% BSA-containing D-PBS (-), and then permeabilized with D-PBS (-) containing 0.1 w/v% Triton X-100 (-) for 5 minutes. Subsequently, after washed with 0.1% BSA-containing D-PBS (-), the cells were reacted with each primary antibody overnight at 4°C. Next, the cells were washed three times with 0.1% BSA-containing D-PBS (-), and reacted with a secondary antibody (anti-rabbit or anti-mouse one), which had been diluted 200-fold, for 60 minutes at room temperature. Then, the cells were washed three times with 0.1% BSA-containing D-PBS (-), and reacted with 1 µg/mL DAPI (manufactured by Dojindo), a nuclear staining reagent, for 5 minutes. After that, the cells were washed three times with D-PBS (-), and analyzed with an Operetta High-Content Imaging System (manufactured by PerkinElmer). The antibodies used in the immunostaining are shown in Table 1.

### <Condition A for inducing differentiation into iBMELCs>

Coating agent: 100 µg/mL Fibronectin, 400 µg/mL Collagen type IV, and 10 µg/mL LN511F.
Medium: HE-SFM supplemented with 7.5% B27 (registered trademark) supplement, 1 µM A 83-01, and 20 ng/mL FGF2.

### [Table 1]

**Table 1**

| Targets | Manufacturer | Catalog number | Species | Dilution |
|---|---|---|---|---|
| PECAM1 | Abca m | ab28364 | Rabbit | 1:25 |
| CDH5 | Santa Cruz | sc-9989 | Mouse | 1:25 |
| P-gp | Abca m | ab10333 | Mouse | 1:25 |
| BCRP | Abca m | ab3380 | Mouse | 1:50 |
| ZO-1 | Fisher Scientific | 33-9100 | Mouse | 1:100 |
| Claudin-5 | Fisher Scientific | 35-2500 | Mouse | 1:50 |
| GLUT1 | Fisher Scientific | MA5-11315 | Mouse | 1:50 |
| Anti-Rabbit (Alexa Flour 488) | Fisher Scientific | A-21206 | Donkey | 1:200 |
| Anti-Mouse (Alexa Fluor 568) | Fisher Scientific | A-11004 | Goat | 1:200 |

### (10) Acetylated LDL uptake test

iEPCs induced to differentiate from hiPSCs were subjected to an acetylated LDL uptake test in order to check the properties as EPCs. First, iEPCs (day 14 of differentiation) induced to differentiate from hiPSCs were reacted with acetylated LDL labeled with Dil at a final concentration of 10 µg/mL for 5 hours, and then reacted with Hoechst 33342, a fluorescent label, for 30 minutes, washed 4 times with a culture medium, and analyzed by Operetta High-Content Imaging System.

### (11) Test of examining effect of freezing iEPCs

In order to examine the effect of cryopreserving iEPCs induced to differentiate from hiPSCs, the difference in transendothelial electrical resistance (TEER value) was compared between the presence and absence of freezing. A frozen group, in which iEPCs on day 14 of differentiation were frozen and thawed by the above-described method, and a unfrozen group of unfrozen iEPCs on day 14 of differentiation were induced to differentiate into iBMELCs by the above-described method while using the above-described differentiation-inducing condition A, and the TEER value was then measured. The number of samples in each of the frozen group or the unfrozen group was 3. The relative maximum TEER value of the frozen group was obtained based on the maximum TEER value of the unfrozen group. As used herein, the "maximum TEER value" means the maximum value among the TEER values measured on each day from day 2 after seeding to day 10 after seeding. The TEER values were measured using a Millicell ERS-2 (chopstick type) according to the attached instructions. The volume of culture medium was 600 µL on the apical side and 1800 µL on the basal side.

### (12) To analyze transendothelial electrical resistance value when conditions of inducing differentiation from iEPCs to iBMELCs varied

The barrier function was evaluated by analyzing the TEER value for iBMELCs induced to differentiate from frozen iEPCs by the above method while using each inducing condition in which the coating agent and the medium were each varied. Unless otherwise indicated, the number of samples was 3 for each. The TEER values were measured as described above.

### (11) Test for permeability of fluorescein isothiocyanate-dextran 4 kDa (FD4) or lucifer yellow (LY)

Permeability was tested for iBMELCs induced to differentiate from frozen iEPCs by the above method while using four inducing conditions different from each other and using, as a permeating substance, FD4 or LY, which are each an indicator of permeability through the paracellular pathway. For the four differentiation-inducing conditions, a coating agent containing two components of FBN and COL4 and an HE-SFM medium containing 20 ng/mL of FGF2 were common conditions. In the sample "KSR", the medium additionally contained 7.5% KSR; in the sample "B27", the medium additionally contained 7.5% B27s; in the sample "B27+A 83-01", the medium additionally contained 7.5% B27s and 1 µM A 83-01; and in the sample "B27+A 83-01+LN511F", the medium additionally contained 7.5% B27s and 1 µM A 83-01, and the coating agent further contained 10 µg/mL LN511F. The number of samples was 3 for each. For the permeability test, cells on a Transwell (registered trademark) (12-well, pore size: 1 µm) were used. On day 4 after seeding, the medium was changed to a transport buffer (HBSS containing 10 mM HEPE solution), and the cells were cultured at 37°C for 20 minutes. The transport buffer containing 1 mg/mL FD4 or 300 µM LY was added to the apical side. After incubation at 37°C for 60 minutes, 100 µL of the solution was collected from the basolateral side. The volume of transport buffer was 500 µL on the apical side and 1500 µL on the basal side. The fluorescence intensity of FD4 or LY was measured with a Synergy HTX multimode plate reader (BioTek) and analyzed by Gen5 data analysis software (BioTek).

### (12) To analyze expression level of brain microvascular endothelial cell marker protein

The expression level of each brain microvascular endothelial cell marker protein was analyzed. Samples used were iBMELCs (day 4 after seeding) induced to differentiate from frozen iEPCs by the above method using the above differentiation-inducing condition A, and as a Comparative Example, cells (day 4 after seeding) cultured using an HE-SFM medium with a coating agent containing 100 µg/mL of Fibronectin and 400 µg/mL of Collagen type IV and supplemented with 7.5% KSR and 20 ng/mL FGF2. The number of samples was 3 for each. The protein expression level was analyzed using an Operetta High-Content Imaging System. Cells immunostained as described above were observed under an Operetta microscope, and the fluorescence intensity was calculated by Harmony (registered trademark) high-content analysis software. Relative expression levels of proteins in iBMELCs were determined on the basis of the expression levels of proteins in Comparative Examples.

### (13) To analyze expression level of brain microvascular endothelial cell marker gene

The expression level of each brain microvascular endothelial cell marker gene was analyzed by RT-q PCR. Samples used were iBMELCs (day 4 after seeding; the number of samples: 3) induced to differentiate from frozen iEPCs by the above method using the above differentiation-inducing condition A, cells induced to differentiate by a method modified from the report of Lippmann *et al.* (hereinafter, also referred to as "iBMELCs 2"; and the number of samples was 3), human immortalized BMECs (hereinafter, also referred to as "hCMEC/D3"; the number of samples: 3), and human primary BMECs (hereinafter, also referred to as "hBMECs"; the number of samples: 1). RNA was extracted according to the instructions attached to Agencourt (registered trademark) RNAdvance Tissue Kit (manufactured by Beckman Coulter, Inc.). For reverse transcription, complementary DNA (cDNA) was synthesized using a ReverTra Ace (registered trademark) qPCR RT Master Mix (manufactured by Toyobo Co., Ltd.) according to the attached instructions. RT-q PCR was performed with a KAPA SYBR Fast qPCR Kit (manufactured by Nippon Genetics Co., Ltd.) while using cDNA as a template, and the reaction was carried out according to the attached instructions. The results obtained were corrected using HPRT1 as an internal control. The relative expression levels of mRNA in iBMELCs, iBMELCs 2, or hCMEC/D3 were determined while the expression levels of mRNA in hBMECs were set to 100 as a reference.

As the primers used in RT-q PCR, the sequence of the forward primer of CDH5 is shown in SEQ ID NO: 1 and the sequence of the reverse primer of CDH5 is shown in SEQ ID NO: 2; the sequence of the forward primer of MDR1 is shown in SEQ ID NO: 3 and the sequence of the reverse primer of MDR1 is shown in SEQ ID NO: 4; the sequence of the forward primer of BCRP is shown in SEQ ID NO: 5 and the sequence of the reverse primer of BCRP is shown in SEQ ID NO: 6; the sequence of the forward primer of GLUT1 is shown in SEQ ID NO: 7 and the sequence of the reverse primer of GLUT1 is shown in SEQ ID NO: 8; the sequence of the forward primer of Occludin is shown in SEQ ID NO: 9 and the sequence of the reverse primer of Occludin is shown in SEQ ID NO: 10; the sequence of the forward primer of ZO-1 is shown in SEQ ID NO: 11 and the sequence of the reverse primer of ZO-1 is shown in SEQ ID NO: 12; the sequence of the forward primer of LAT1 is shown in SEQ ID NO: 13 and the sequence of the reverse primer of LAT1 is shown in SEQ ID NO: 14; the sequence of the forward primer of HPRT1 is shown in SEQ ID NO: 15, and the sequence of the reverse primer of HPRT1 is shown in SEQ ID NO: 16; and the sequence of the forward primer of PECAM1 is shown in SEQ ID NO: 17 and the sequence of the reverse primer of PECAM1 is shown in SEQ ID NO: 18.

### (14) To compare electrical resistance value when differentiation-inducing conditions were varied in iEPCs and immortalized cells

TEER values of iEPCs and immortalized cells were compared and examined. Here, used were iBMELCs (on day 4 after seeding) induced to differentiate from frozen iEPCs by the above method and hCMEC/D3 (on day 4 after seeding) induced for differentiation while using the above differentiation-inducing condition A, and as a Comparative Example, cultured iEPCs and hCMEC/D3 (on day 4 after seeding) not induced for differentiation while using an HE-SFM medium with a coating agent containing 100 µg/mL of Fibronectin and 400 µg/mL of Collagen type IV and supplemented with 7.5% KSR and 20 ng/mL FGF2. The number of samples was 3 for each. The TEER values were measured as described above, and the maximum TEER values were compared.

### (15) To analyze function of P-gp

Substrate uptake was tested on iBMELCs (day 4 after seeding) induced to differentiate from frozen iEPCs by the above method using the above differentiation-inducing condition A in order to analyze the function of P-glycoprotein (P-gp) as an efflux transporter. For the substrate uptake test, cells on a Transwell (registered trademark) (12-well, pore size: 1 µm) were used. The substrate used was rhodamine 123. The culture medium was removed and the cells were pre-incubated in a transport buffer for 15 minutes at 37°C. Note that the number of samples was 3. The cells were incubated, in the presence or absence of 10 µM cyclosporine A (CsA), an inhibitor of P-gp, in a transport buffer containing 10 µM rhodamine 123 for 60 minutes at 37°C. The cells were then washed three times with PBS and lysed in PBS containing 5 w/v% Triton X-100. The fluorescence intensities of rhodamine 123 were measured with a Synergy HTX multimode plate reader and analyzed by Gen5 data analysis software.

### 2. Results and Discussion

### (1) Results of checking properties of iEPCs

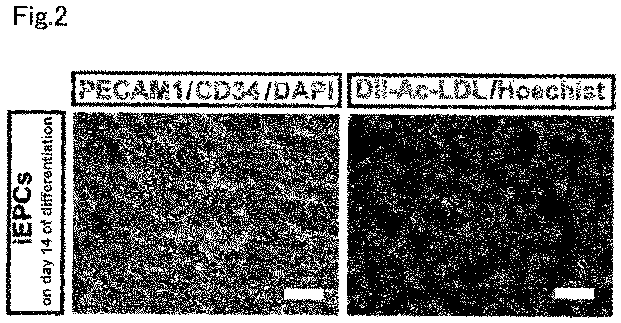

Fig. 2 is images illustrating the results of checking the properties of iEPCs on day 14 of differentiation. In Fig. 2, the image on the left side of the drawing shows the result of analyzing protein expression by immunostaining, and the image on the right side of the drawing shows the result of an acetylated LDL uptake test. In both images, a scale bar of 100 µm is shown. The results shown in Fig. 2 have demonstrated that PECAM1 and CD34, which are vascular endothelial progenitor cell markers, are expressed, and that the cells have an acetylated LDL uptake function, which is one of the functions of vascular endothelial progenitor cells. That is, the cells differentiated and purified by the above method were found to have the properties of vascular endothelial progenitor cells.

### (2) Results of examining effect of cryopreserving iEPCs

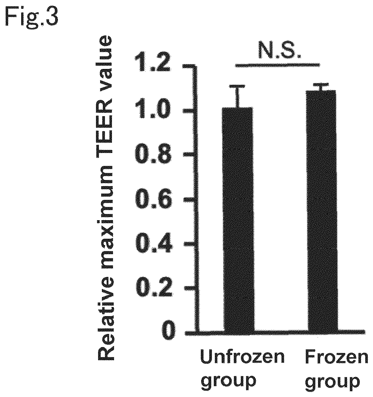

Fig. 3 is a graph illustrating comparison of electric resistance values between unfrozen iEPCs and frozen iEPCs. In Fig. 3, the ordinate represents the relative maximum TEER value (Ω × cm²) while the maximum TEER value of the unfrozen group is set to 1.0 as a reference. The results shown in Fig. 3 have demonstrated that no significant difference in the maximum TEER value is present between the unfrozen group and the frozen group, and that the frozen iEPCs have a barrier function equivalent to that of the unfrozen iEPCs. Therefore, iEPCs induced to differentiate from hiPSCs were found to be able to be cryopreserved to prepare an iEPCs stock, and the iEPCs stock can thus be utilized for preparing iBMELCs.

### (3) Results of examining conditions for inducing differentiation from iEPCs to iBMELCs

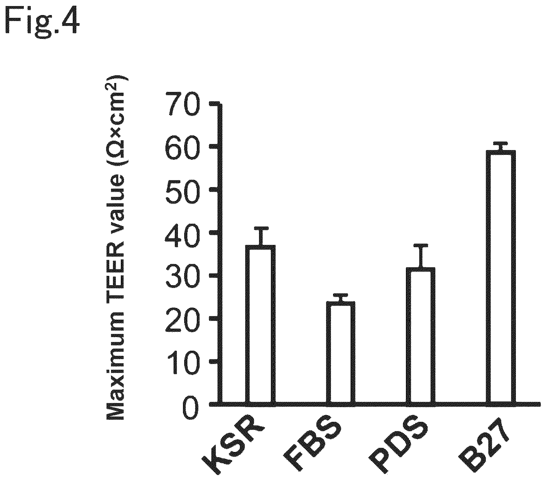

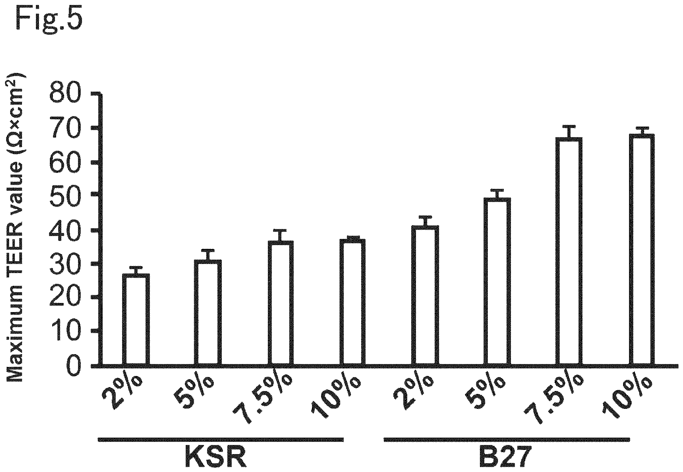

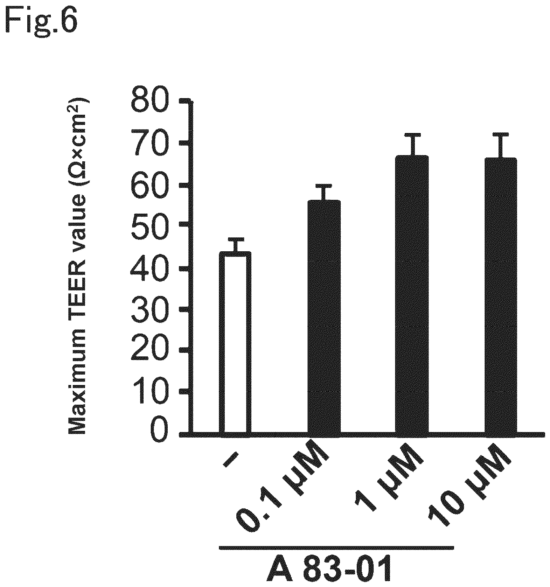

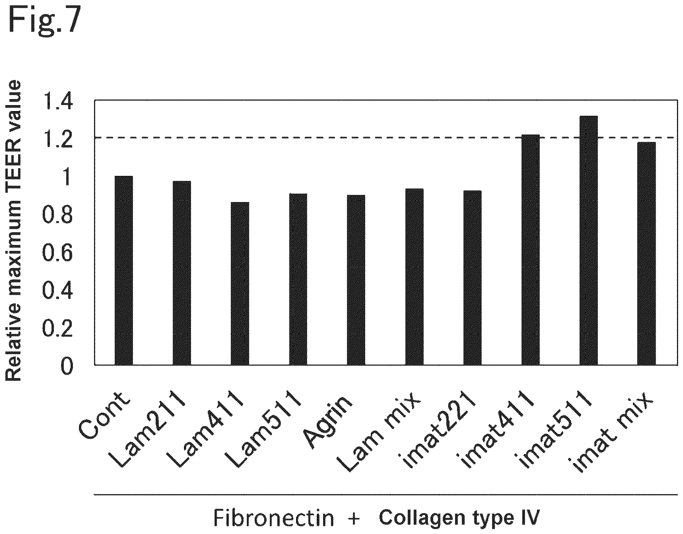

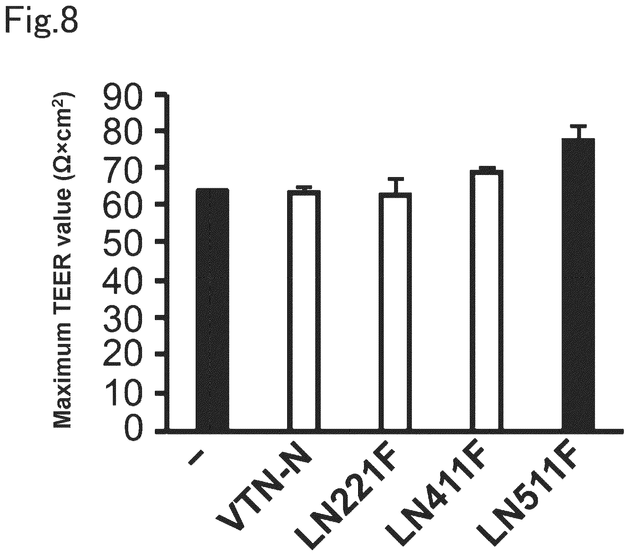

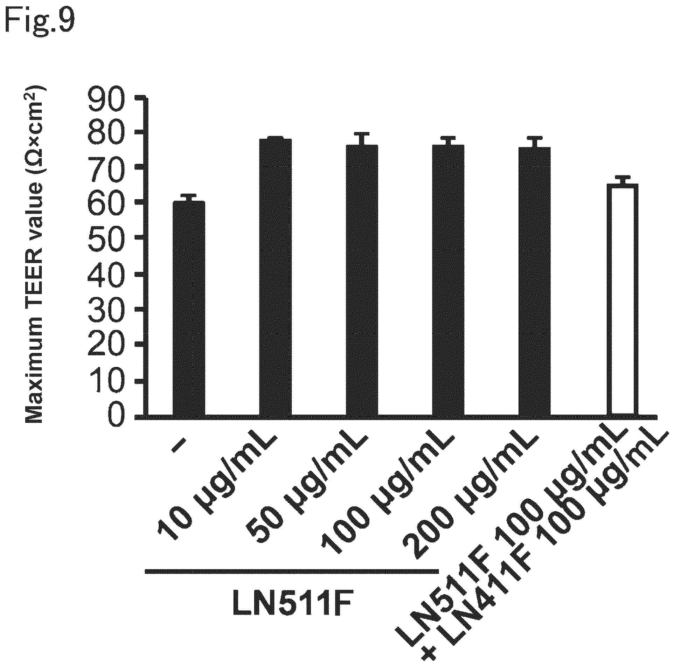

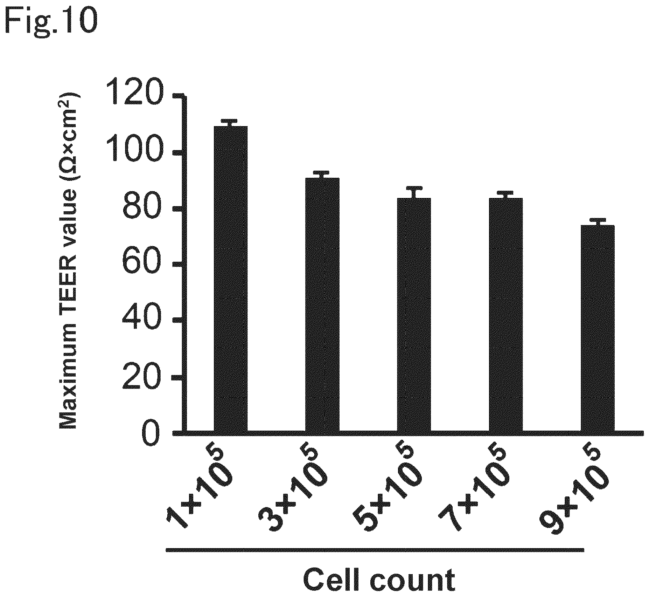

Figs. 4 to 10 are graphs illustrating the results of examining conditions for inducing differentiation from iEPCs into iBMELCs. As the differentiation-inducing conditions, Fig. 4 shows the kind of medium component, Fig. 5 shows the concentration of the medium component, Fig. 6 shows the concentration of A 83-01, Figs. 7 and 8 show the kind of coating component, Fig. 9 shows the concentration of LN511F, and Fig. 10 shows the cell count as the examination results. In Figs. 4 to 6 and 8 to 10, the ordinate represents the maximum TEER value (Ω × cm²). In Fig. 7, the ordinate represents the relative maximum TEER value of each sample when the maximum TEER value (Ω × cm²) of control (only two components of FBN and COL4) is set to 1.0 as a reference.

Fig. 4 shows the results of examining the cases where 5% of each of KSR, FBS, PDS, or B27s was added to an HE-SFM medium containing two components of FBN and COL4 as a coating agent and 20 ng/mL FGF2. The results shown in Fig. 4 have revealed that the barrier function of iBMELCs can be particularly improved by adding B27s to the medium to induce differentiation.

Fig. 5 shows the results of examining the cases where 2 to 10% of KSR or B27s was added to an HE-SFM medium containing two components of FBN and COL4 as a coating agent and 20 ng/mL FGF2. The results shown in Fig. 5 have revealed that as the content of B27s in the medium increases, the barrier function of iBMELCs is improved, and in the case of the content of 7.5%, a high barrier function equivalent to that in the case of the content of 10% is exhibited. In addition, it has been found that the addition of B27s improves the barrier function of iBMELCs more than the addition of KSR at any content.

Fig. 6 shows the results of examining the cases where A 83-01 was added at 0.1 µM, 1 µM, or 10µM to an HE-SFM medium containing two components of FBN and COL4 as a coating agent, 20 ng/mL FGF2, and 7.5% B27s. The results shown in Fig. 6 have revealed that when A 83-01 was added, the barrier function of iBMELCs was higher than that when A 83-01 was not added. In addition, the case of the A 83-01 content of 1 µM was found to exhibit a high barrier function equivalent to the case of the content of 10 µM.

Fig. 7 shows the results of examining (number of samples: 1) the cases where 10 µg/mL of each component was added to two components of FBN and COL4 as a coating agent. Note that the culture medium used was HE-SFM supplemented with 7.5% B27s, 1 µM A 83-01, and 20 ng/mL FGF2. The results shown in Fig. 7 have revealed that the barrier function of iBMELCs can be particularly improved by adding LN511F to the coating agent.

Fig. 8 shows the results of examining the cases where 10 µg/mL of each component was added to two components of FBN and COL4 as a coating agent. Note that the culture medium used was HE-SFM supplemented with 7.5% B27s, 1 µM A 83-01, and 20 ng/mL FGF2. The results shown in Fig. 8 have revealed that the barrier function of iBMELCs can be particularly improved by adding LN511F to the coating agent.

Fig. 9 shows the results of examining the cases where LN511F was added at 10 µg/mL, 50 µg/mL, 100 µg/mL, or 200 µg/mL to two components of FBN and COL4 as a coating agent. In addition, the results of examination are shown in which LN411F and LN511F were each added at 100 µg/mL to the two components of FBN and COL4. Note that the culture medium used was HE-SFM supplemented with 7.5% B27s, 1 µM A 83-01, and 20 ng/mL FGF2. The results shown in Fig. 9 have revealed that inclusion of 10 µg/mL LN511F in the coating agent results in a high barrier function equivalent to that in the case of the content of 200 µg/mL. In addition, the barrier function of iBMELCs was found to be more effectively improved in the case of using a coating layer containing only LN511F in addition to the two components of FBN and COL4 than in the case of using a coating layer containing LN411F and LN511F used in combination with the two components of FBN and COL4.

Fig. 10 shows the results of examining the cases where the number of iEPCs seeded was varied under the differentiation-inducing condition A. The results shown in Fig. 10 have demonstrated that since the maximum TEER value when the number of cells was 1 × 10⁵ cells/well was the highest, a low cell density setting can further improve the barrier function of iBMELCs.

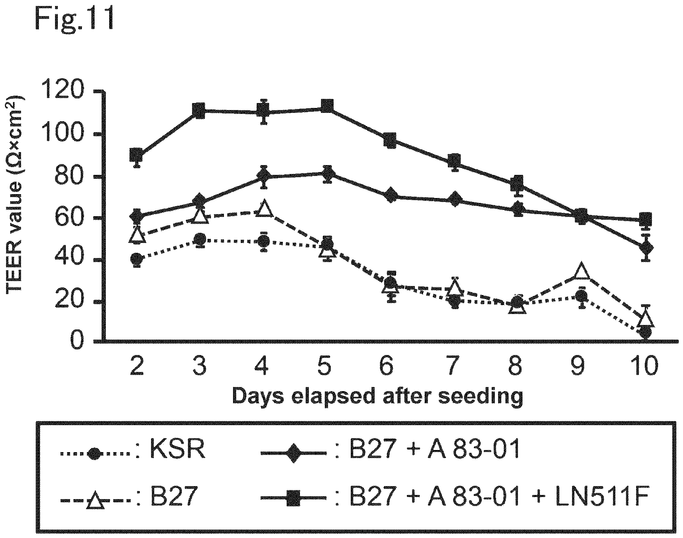

Fig. 11 is a graph indicating a change in TEER value over time under each differentiation-inducing condition. In Fig. 11, the ordinate represents the TEER value (Ω×cm²), and the abscissa represents the number of days elapsed after seeding. For the four differentiation-inducing conditions used in Fig. 11, a coating agent containing two components of FBN and COL4 and an HE-SFM medium containing 20 ng/mL of FGF2 were common conditions. In the sample "KSR", the medium additionally contained 7.5% KSR; in the sample "B27", the medium additionally contained 7.5% B27s; in the sample "B27+A 83-01", the medium additionally contained 7.5% B27s and 1 µM A 83-01; and in the sample "B27+A 83-01+LN511F", the medium additionally contained 7.5% B27s and 1 µM A 83-01, and the coating agent further contained 10 µg/mL LN511F. The results shown in Fig. 11 have revealed that iBMELCs induced to differentiate using a coating agent containing LN511F had a higher TEER value over a long period of time than iBMELCs induced to differentiate using a coating agent free of LN511F. That is, it has been demonstrated that iBMELCs induced to differentiate using a coating agent containing LN511F has a higher barrier function over a long period of time than iBMELCs induced to differentiate using a coating agent free of LN511F.

### (4) Permeability test results

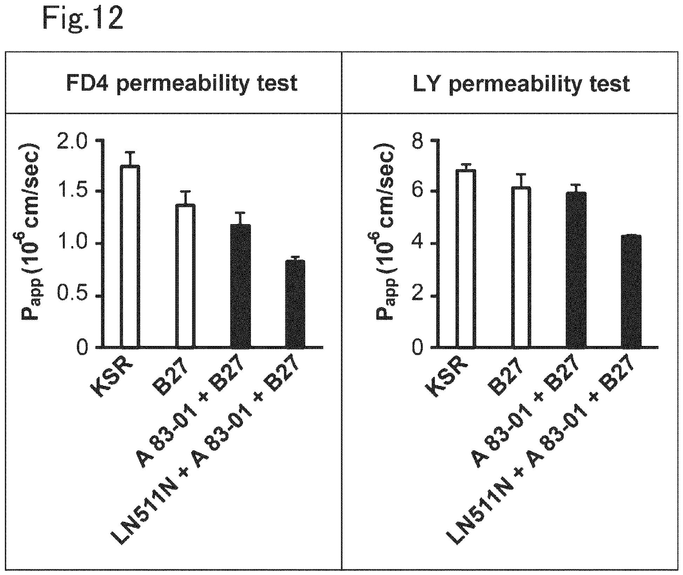

Fig. 12 is graphs illustrating the results of a permeability test of FD4 or LY under each differentiation-inducing condition. In Fig. 12, the ordinate represents the permeability coefficient Pₐₚₚ (10⁻⁶ cm/sec). The four differentiation-inducing conditions used in Fig. 12 are the same as the differentiation-inducing conditions used in the analysis of the change in TEER value over time. The results shown in Fig. 12 have revealed that even in the case of using, as a permeating target, any of FD4 or LY, which are each an indicator of permeability through a paracellular pathway, iBMELCs induced to differentiate using a coating agent containing LN511F had a lower permeability coefficient than iBMELCs induced to differentiate using a coating agent free of LN511F. That is, it has been demonstrated that iBMELCs induced to differentiate using a coating agent containing LN511F have a higher barrier function than iBMELCs induced to differentiate using a coating agent free of LN511F.

The results of the TEER value measurement and the permeability test have suggested that iBMELCs induced to differentiate using a coating agent containing LN511F in addition to FBN and COL4 have a higher barrier function than iBMELCs induced to differentiate using a coating agent free of LN511F. Therefore, it has been found that iBMELCs produced by a production method including a culturing step of culturing iEPCs by using LN511F, FBN, and COL4 are useful as a model of human BBB. In addition, the differentiation induction using the B27s-containing medium can be found to further improve the barrier function of iBMELCs as compared to the case of inducing differentiation using the B27s-free medium. Furthermore, when A 83-01 was added, the barrier function of iBMELCs was higher than that when A 83-01 was not added.

### (5) Results of analyzing expression of brain microvascular endothelial cell marker protein

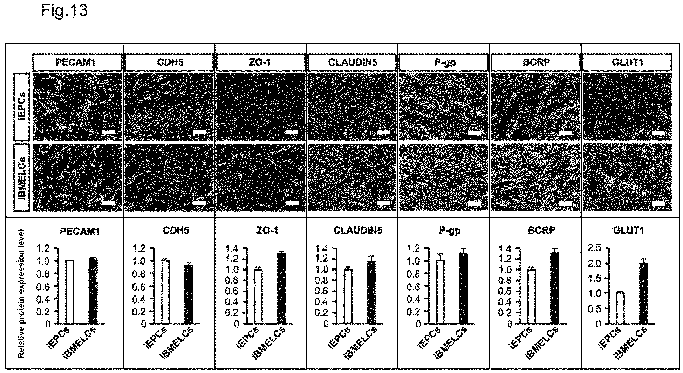

Fig. 13 is images and graphs illustrating the results of analyzing the expression of each protein by immunostaining and the results of analyzing the expression level of each protein by Operetta High-Content Imaging System. The upper side of the drawing of Fig. 13 shows each image obtained by immunofluorescence staining for each BMECs marker together with a scale bar of 50 µm, and the lower side of the drawing of Fig. 13 shows the relative protein expression level. The relative protein expression level is designated as a relative value when the protein expression level in cells of Comparative Example (described as "iEPCs" in Fig. 13) is set to 1.0 as a reference. The results of analyzing the protein expression by immunostaining revealed the following. Specifically, since PECAM1 and CDH5 as representative vascular endothelial cell markers were localized on the cell membrane of iBMELCs, it has been suggested that iBMELCs have properties as vascular endothelial cells. In addition, iBMELCs have higher expression levels of BMECs markers (e.g., ZO-1 and CLAUDIN5 as tight junction markers, P-gp and BCRP as drug efflux transporter markers, GLUT1 as a glucose transporter marker) than those of the cells of Comparative Example. This has suggested that iBMELCs produced by the differentiation-inducing method described above have strong characteristics as brain microvascular endothelial cells.

### (6) Results of analyzing expression of brain microvascular endothelial cell marker gene

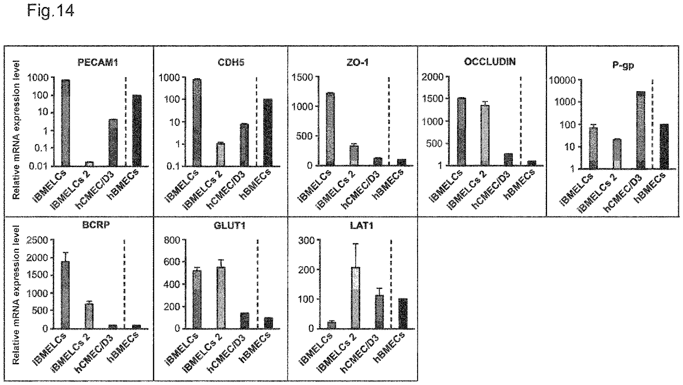

Fig. 14 is graphs illustrating the results of analyzing each gene expression by the RT-q PCR method. The ordinate in Fig. 14 represents the relative mRNA expression level when the mRNA expression level in human primary BMECs (hBMECs) is set to 100 as a reference. The results shown in Fig. 14 have revealed that iBMELCs exhibit a significantly higher expression level of each BMECs marker including PECAM1, CDH5, ZO-1, OCCLUDIN, and BCRP than cells induced to differentiate by a method modified from the report by Lippmann *et al.* (iBMELCs 2), hBMECs, or human immortalized BMECs (hCMEC/D3). In addition, iBMELCs exhibited a significantly higher expression level of GLUT1 than hBMECs or hCMEC/D3, and exhibited the expression level equivalent to that of iBMELCs 2. Further, iBMELCs were found to express P-gp at a level equivalent to that of hBMECs.

The results of protein expression analysis and gene expression analysis have suggested that iBMELCs produced by the differentiation-inducing method described above have properties as brain microvascular endothelial cells and are very similar to BMECs *in vivo.* Therefore, iBMELCs produced by the differentiation-inducing method described above are found to be useful as a model of human BBB by exhibiting properties similar to those of BMECs *in vivo.*

### (7) Results of comparing electrical resistance values when differentiation-inducing conditions were varied in iEPCs and immortalized cells

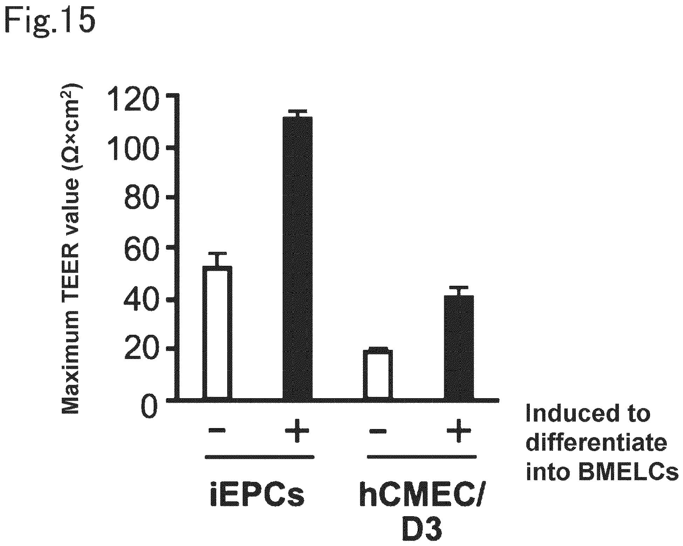

Fig. 15 is a graph illustrating comparison of electrical resistance values in iEPCs and immortalized cells. The ordinate in Fig. 15 represents the maximum TEER value (Ω × cm²). The "+" indicates a sample induced to differentiate using the above-described differentiation-inducing condition A. The "-" indicates, as a Comparative Example, a sample cultured under the above-described conditions different from the differentiation-inducing condition A. The results shown in Fig. 15 have revealed that in either case of using iPS cells or immortalized cells, cells induced to differentiate by the above method using the differentiation-inducing condition A showed a higher TEER value. That is, it has been demonstrated that brain microvascular endothelial-like cells having a barrier function that mimic brain microvascular endothelial cells can be produced by inducing differentiation by the above-described culture method in both cases of iPS cell-derived cells and immortalized cell-derived cells. In addition, the results shown in Fig. 15 have verified that iBMELCs having a higher barrier function can be produced by inducing differentiation using iPS cells when compared to the case of inducing differentiation using immortalized cells.

### (8) Results of analyzing function of P-gp

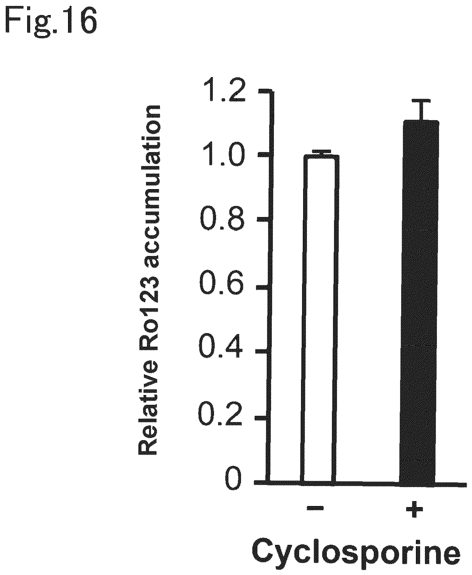

Fig. 16 is a graph illustrating the results of analyzing the function of P-glycoprotein (P-gp). In Figs. 9 and 16, the ordinate represents the relative substrate accumulation when the substrate accumulation in the absence of cyclosporine is set to 1.0 as a reference. The "-" indicates a sample in the absence of cyclosporine, and the "+" indicates a sample in the presence of cyclosporine. The results shown in Fig. 16 have verified that the accumulation of substrate in iBMELCs induced to differentiate by the above method is larger in the presence of cyclosporine, an efflux transporter inhibitor, than in the absence of such an inhibitor. This has revealed that iBMELCs induced to differentiate by the above method have a function of P-gp.

### 3. Conclusion

The above results have demonstrated that the differentiation induction using the above method can be used to produce brain microvascular endothelial-like cells having properties as vascular endothelial cells and further mimicking (more similar to) brain microvascular endothelial cells *in vivo.*

### 4. Co-culture of iEPCs and iBPCs

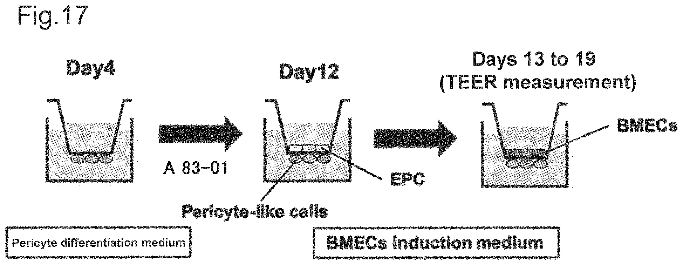

Fig. 17 is a scheme for explaining an outline of co-culture of iEPCs and iBPCs. Fig. 17 schematically illustrates an outline of a method for inducing differentiation of human iPS cells (hiPSCs) into human iPS cell-derived brain pericytes (hereinafter, also referred to as "iBPCs") and co-culturing the iBPCs with the above-described iEPCs. As described later, iBPCs were seeded on the membrane on the back surface of a cell culture insert, A 83-01 was added to the culture medium on day 8 of differentiation induction, and the cells were then cultured in a pericyte differentiation medium. Thereafter, iEPCs were seeded on the front side of the cell culture insert on day 12 of differentiation induction, and co-cultured in a BMECs differentiation medium. The barrier function was evaluated by measuring and analyzing the TEER value from day 13 to day 19 of differentiation induction, that is, from day 1 to day 7 of co-culture.

### (1) Materials and others

First, hiPSCs used for inducing differentiation into iBPCs were 802-3G strain (obtained from REPROCELL Inc.). For Matrigel GFR coating used at the time of maintaining and culturing the hiPSCs, Matrigel GFR (manufactured by Corning Incorporated) was diluted 30 times with a human iPS cell maintenance medium on ice, and allowed to stand at 37°C for 30 minutes or longer to prepare the coating. The hiPSCs were cultured on a culture dish coated with VTN-N while using mTeSR plus medium (manufactured by StemCell Technologies) as a culture medium. A detachment solution used when the 802-3G strain was subcultured was 0.5 M EDTA (pH 8.0) or TrypLE Select (manufactured by Thermo Fisher Scientific). When TrypLE Select was used for detachment, 10 µM of Y-27632 was added immediately after subculture, and the next day the culture medium was changed. Note that the VTN-N coating on the culture dish was prepared by diluting VTN-N (manufactured by Thermo Fisher Scientific) to 1 µg/cm² with D-PBS (-), and then allowed to stand at 37°C for 1 to 2 hours to prepare the coating.

### (2) Co-culture method

iBPCs were used which were induced to differentiate from hiPSCs until day 4 of differentiation by the method described later. As a coating for the culture dish, three components of 100 µg/mL Fibronectin, 400 µg/mL Collagen type IV, and 10 µg/mL LN511F were applied onto the membrane on the apical side of the cell culture insert. As a coating for the culture dish, 1 µg/cm² of VTN-N was applied onto the membrane on the basal side of the cell culture insert. The cell culture insert was turned over on a 10-cm culture dish. Next, iBPCs (day 4 of differentiation) were seeded at 1.2 × 10⁴ cells/cm² on the membrane, and then cultured for 2 hours in an EGM-2 medium containing 10 µM Y-27632, 50 ng/mL PDGF-BB, and 1 µM A 83-01. Thereafter, the cell culture insert seeded with iBPCs were placed in a 12-well companion plate. The cells on the apical side or the basal side were then cultured for 2 hours in 600 µL or 1800 µL, respectively, of an EGM-2 medium containing 10 µM Y-27632 and 50 ng/mL PDGF-BB. From day 5 of differentiation, the cells were cultured in an EGM-2 medium containing 50 ng/mL of PDGF-BB. From day 8 of differentiation, the cells were cultured in an EGM-2 medium containing 50 ng/mL PDGF-BB, and 1 µM A 83-01. During this time, the medium was changed every day. On day 12 of differentiation, frozen iEPCs (day 14 of differentiation) were seeded at 1 × 10⁵ cells/cm² on the apical side, and cultured until day 19 of differentiation in an HE-SFM medium containing 7.5% B27 supplement, 1 µM A 83-01, and 20 ng/mL FGF2, thereby inducing differentiation into brain microvascular-like cells. During this time, the medium was changed every day.

### (3) To analyze transendothelial electrical resistance value in iBMELCs in presence or absence of co-culture

The barrier function was evaluated by analyzing the TEER value for iBMELCs induced to differentiate under conditions where frozen iEPCs and iBPCs were co-cultured by the above method. Two-way Repeated-Measures ANOVA was used for the analysis. In addition, TEER values were similarly measured for iBMELCs when frozen iEPCs were cultured alone by the above method. The number of samples was 3 for each. The TEER values were measured using a Millicell ERS-2 (chopstick type) according to the attached instructions. The volume of culture medium was 600 µL on the apical side and 1800 µL on the basal side.

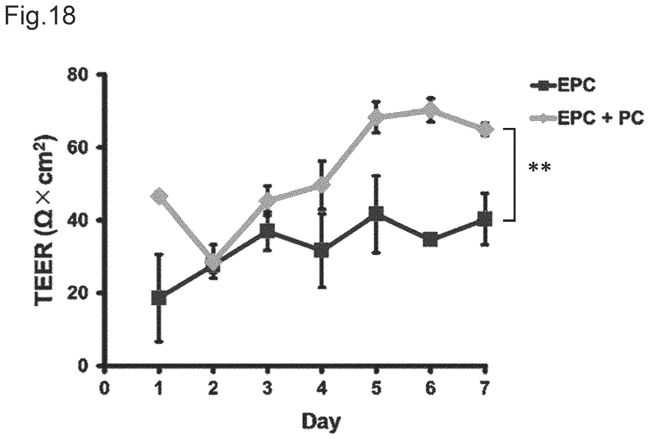

Fig. 18 is a graph illustrating a change in TEER value over time in iBMELCs in the presence or absence of co-culture. The ordinate in Fig. 18 represents the TEER value (Ω × cm²). The abscissa represents the number of days elapsed after co-culture (days in the case of culturing iEPCs alone without co-culture corresponds to the days in the case of co-culture). In Fig. 18, the sample co-cultured with iBPCs is denoted as "EPC + PC", and the sample cultured with iEPCs alone without co-culture with iBPCs is denoted as "EPC".

The results shown in Fig. 18 have indicated the following. In the step of culturing iEPCs, the TEER values of iBMELCs co-cultured with iBPCs were compared to the TEER values of iBMELCs obtained by culturing iEPCs alone. A significant difference was detected at the 1% level by Two (or more)-way Repeated-Measures ANOVA, indicating that the TEER value increased over time. Therefore, the co-culture with iBPCs has been found to further enhance the tight junction function of iBMELCs.

### 5. How A 83-01 affected induced differentiation into iBPCs.

How A 83-01 affected induced differentiation from hiPSCs into iBPCs was examined.

### (1) Induced differentiation

The above-described hiPSCs were used and induced to differentiate into iBPCs. Human iPS cells cultured in mTeSR plus medium were detached by using TrypLE Select, seeded on a VTN-N-coated culture dish at a density of 2 × 10⁴ cells/cm², and then cultured in mTeSR plus medium containing 10 µM of Y-27632 (manufactured by Focus Biomolecules) for 24 hours. Thereafter, the cells were cultured for 1 day in modified DMEM/F12 medium (DMEM/F12 containing 0.1% chemically defined lipid concentrate, 0.1 × insulin-transferrin-selenium (from Thermo Fisher Scientific), 2 mM GlutaMAX (from Sigma-Aldrich), 450 mM 1-thioglycerol (from Sigma-Aldrich), 50 µg/mL of L-ascorbic acid phosphate magnesium salt n-hydrate, and 1 × penicillin-streptomycin solution) supplemented with 5 µM CHIR-99021 (manufactured by Focus Biomolecules). Next, the cells were cultured for 1 day in modified DMEM/F12 medium containing 50 ng/mL of FGF2. Then, the cells were cultured for 2 days in EGM-2 medium (EGM-2 basal medium containing 2% FBS, 0.2 µg/mL hydrocortisone, 10 ng/mL FGF2, 20 ng/mL R3-IGF-1, 1 µg/mL ascorbic acid, 5 ng/mL hEGF, gentamicin, and amphotericin B) supplemented with 50 ng/mL PDGF-BB and 0.5 ng/mL VEGF. During this time, the medium was changed every day. On day 4, cells treated with 10 µM Y-27632 for 1 hour were dissociated using TrypLE Select, subcultured on a new VTN-N-coated culture dish at 1.25 × 10⁴ cells/cm², and then induced to differentiate in an EGM-2 medium containing 50 ng/mL PDGF-BB. During this time, the medium was changed every day. On day 8 of differentiation, the cells were induced to differentiate in an EGM-2 medium containing 50 ng/mL PDGF-BB, and 1 µM A 83-01. During this time, the medium was changed every day. In the following description, iBPCs induced to differentiate by adding A 83-01 to the culture medium are also referred to as "X (+)", and iBPCs induced to differentiate without adding A 83-01 to the culture medium are also referred to as "X (-)".

### (2) Immunostaining analysis

iBPCs (X(+) and X(-)) induced to differentiate from hiPSCs by the above method were analyzed by immunostaining in order to check the properties as BPCs. PDGFR-β, NG2, and α-SMA antibodies were used for this immunostaining. The cells on a 96 well plate were washed 3 times with 0.1% BSA-containing D-PBS (-), fixed in 4% paraformaldehyde for 15 minutes, washed again 3 times with 0.1% BSA-containing D-PBS (-), and then permeabilized with D-PBS (-) containing 0.1 w/v% Triton X-100 (-) for 5 minutes. Subsequently, after washed with 0.1% BSA-containing D-PBS (-), the cells were reacted with each primary antibody overnight at 4°C. Next, the cells were washed three times with 0.1% BSA-containing D-PBS (-), and reacted with a secondary antibody (anti-rabbit one), which had been diluted 200-fold, for 60 minutes at room temperature. Then, the cells were washed three times with 0.1% BSA-containing D-PBS (-), and reacted with 1 µg/mL DAPI (manufactured by Dojindo), a nuclear staining reagent, for 5 minutes. After that, the cells were washed three times with D-PBS (-), and then analyzed with an Operetta High-Content Imaging System (manufactured by PerkinElmer). The antibodies used in the immunostaining are shown in Table 2. Note that in Table 2, "Target" represents an antibody name; "Source" represents a name of manufacturer of the antibody; "Catalog number" indicates an antibody catalog number; "Species" indicates the origin of the antibody; and "Dilution" indicates a dilution factor in terms of volume.

### [Table 2]

**Table 2**

| Target | Source | Catalog number | Species | Dilution |
|---|---|---|---|---|
| PDGFR-*β* | Abeam | ab32570 | Rabbit | 1 : 100 |
| NG2 | Sigma-Aldrich | AB5320 | Rabbit | 1 : 100 |
| *α* -SMA | Abeam | ab5694 | Rabbit | 1 : 100 |
| Anti-rabbit (Alexa Fluor 488) | Thermo Fisher Scientific | A-21206 | Donkey | 1 : 200 |

### (3) To analyze expression level of brain pericyte marker gene

The expression level of each brain pericyte marker gene was analyzed by RT-q PCR. Samples used were iBPCs (X (+)) on day 12 of differentiation as induced to differentiate by adding A 83-01 in the above method, iBPCs (X (-)) on day 12 of differentiation as induced to differentiate without adding A 83-01 in the above method, and human primary pericytes (hereinafter, also referred to as "hPCs"). The number of samples was 3 for each. RNA was extracted according to the instructions attached to Agencourt (registered trademark) RNAdvance Tissue Kit (manufactured by Beckman Coulter, Inc.). For reverse transcription, complementary DNA (cDNA) was synthesized using a ReverTra Ace (registered trademark) qPCR RT Master Mix (manufactured by Toyobo Co., Ltd.) according to the instructions attached to the device. RT-q PCR was performed with a KAPA SYBR Fast qPCR Kit (manufactured by Nippon Genetics Co., Ltd.) while using cDNA as a template, and the reaction was carried out according to the instructions attached to the device. The results obtained were corrected using HPRT1 as an internal control. Relative expression levels of mRNA in X (+) and X (-) were determined while the expression level of mRNA in hPCs was set to 1 as a reference. One-way ANOVA and Tukey's test were used for analysis.

As primers used in RT-q PCR, the sequence of the forward primer of PDGFR-β is shown in SEQ ID NO: 19 and the sequence of the reverse primer of PDGFR-β is shown in SEQ ID NO: 20; the sequence of the forward primer of NG2 is shown in SEQ ID NO: 21 and the sequence of the reverse primer of NG2 is shown in SEQ ID NO: 22; the sequence of the forward primer of α-SMA is shown in SEQ ID NO: 23 and the sequence of the reverse primer of α-SMA is shown in SEQ ID NO: 24; and the sequence of the forward primer of HPRT1 is shown in SEQ ID NO: 15 and the sequence of the reverse primer of HPRT1 is shown in SEQ ID NO: 16.

### (4) To analyze cell migration ability and proliferation potential

Samples used were iBPCs (X (+)) induced to differentiate by adding A 83-01 in the above method and iBPCs (X (-)) induced to differentiate without adding A 83-01 in the above method. The number of samples was 1 for each. iBPCs (day 12 of differentiation) induced to differentiate on a 6-well plate by the above method were longitudinally scratched using a 10-µL pipette tip. Next, the cells were cultured using an EGM-2.medium containing 50 ng/mL PDGF-BB. At the time of the culture, 0.1 to 10 µM of A 83-01 was further added to the culture medium of iBPCs (X (+)) induced to differentiate by adding A 83-01, and no A 83-01 was added to the culture medium of iBPCs (X (-)) induced to differentiate without adding A 83-01. How the cells migrated 0, 12, and 24 hours of culture after cell scratching was checked by bright field observation, and the scratched area was calculated by Image J.

### (5) Results and Discussion

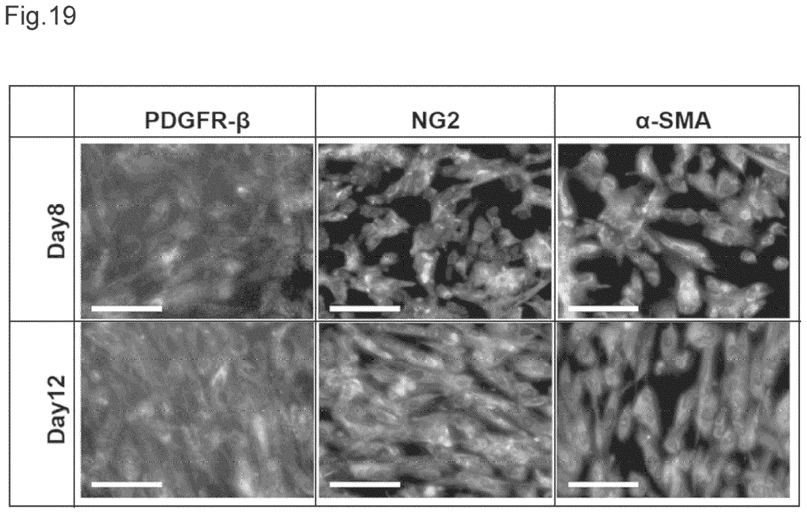

Fig. 19 is images illustrating the results of immunostaining each brain pericyte marker protein. Fig. 19 shows the results of immunostaining on day 8 of differentiation and day 12 of differentiation for iBPCs (X (-)) induced to differentiate without adding A 83-01. In each image, a scale bar of 100 µm is shown. The results shown in Fig. 19 have revealed that the protein expression of PDGFR-β, NG2, and α-SMA became stronger over time from day 8 of differentiation to day 12 of differentiation. Incidentally, it is known that expression of α-SMA is weak in brain pericytes *in vivo.*

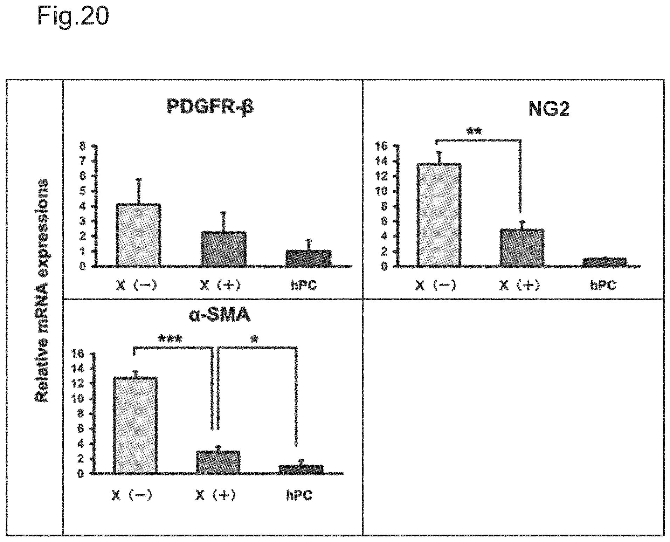

Fig. 20 is graphs illustrating the results of analyzing the expression level of each brain pericyte marker gene. The ordinate in Fig. 20 represents the relative mRNA expression level when the mRNA expression level in human primary pericytes is set to 1 as a reference. In Fig. 20, "*" indicates that a significant difference was observed at the 5% level by multiple comparison (Tukey's test) in one-way analysis of variance; "**" indicates that a significant difference was observed at the 1% level by multiple comparison (Tukey's test) in one-way analysis of variance; and "***" indicates that a significant difference was observed at the 0.1% level by multiple comparison (Tukey's test) in one-way analysis of variance. The results shown in Fig. 20 have revealed the following. Specifically, addition of A 83-01 to the culture medium for inducing differentiation into iBPCs was found to be able to suppress the gene expression of α-SMA to the same extent as that of human primary pericytes.

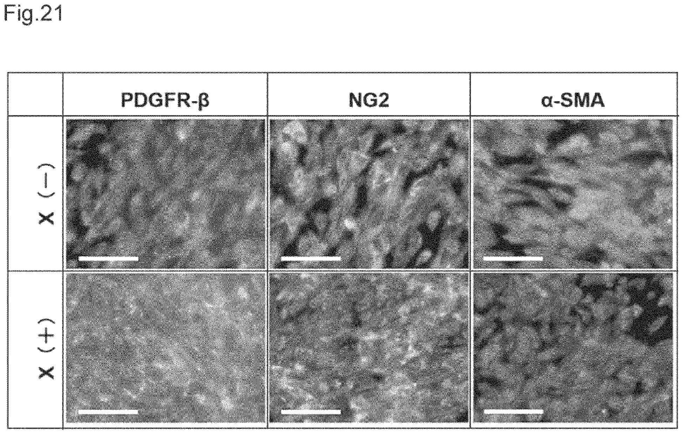

Fig. 21 is images illustrating the results of immunostaining each brain pericyte marker protein. In Fig. 21, a 100-µm scale bar is shown along with each image obtained by immunofluorescence staining for each marker.

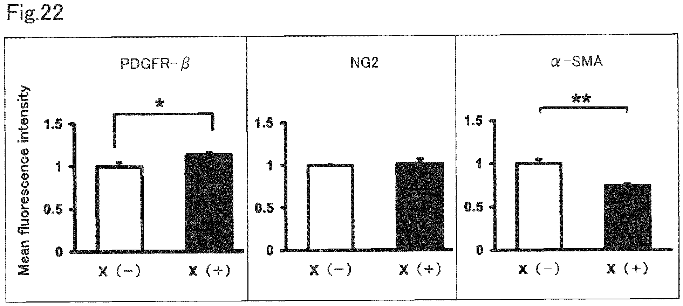

Fig. 22 is graphs illustrating the results of analyzing expression of each brain pericyte marker protein. Fig. 22 shows the results of analyzing the expression level of each marker protein by Operetta High-Content Imaging System. The ordinate in Fig. 22 represents the relative value of the average fluorescence intensity when the average fluorescence intensity of the marker protein in iBPCs (X (-)) induced to differentiate without adding A 83-01 is set to 1 as a reference. In Fig. 22, "*" indicates that a significant difference was observed at the 5% level in Student's t-test, and "**" indicates that a significant difference was observed at the 1% level in Student's t-test.

The results shown in Fig. 21 and 22 have revealed the following. Specifically, addition of A 83-01 to the culture medium for inducing differentiation into iBPCs was found to be able to suppress the protein expression of α-SMA. Also, the addition of A 83-01 did not significantly affect the expression of PDGFR-β or NG2. Therefore, the results shown in Figs. 20 to 22 have demonstrated that by inducing differentiation from pluripotent stem cells to brain pericytes while using a medium containing A 83-01, the cells can further mimic (can be more similar to) brain pericytes *in vivo.*

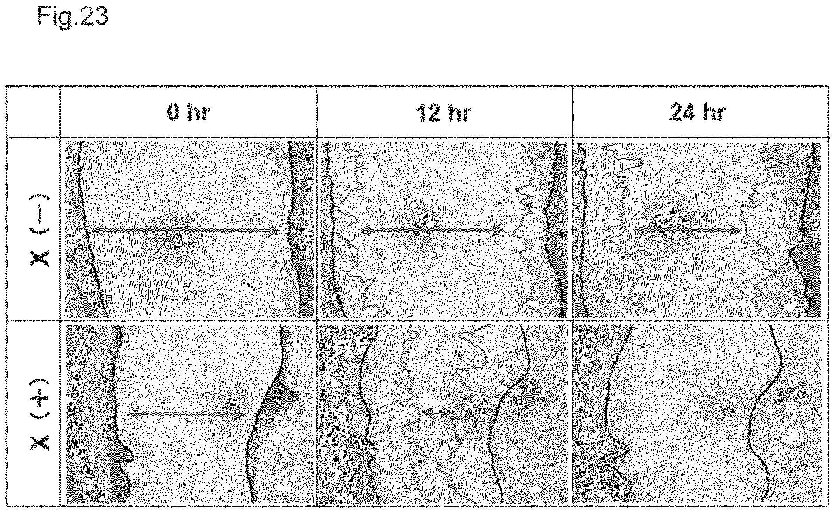

Fig. 23 is microscopic images showing the cell migration ability and proliferation potential of iBPCs. Each bidirectional arrow in Fig. 23 indicates the distance of the cell scratch portion, in other words, the area where no cell is present.

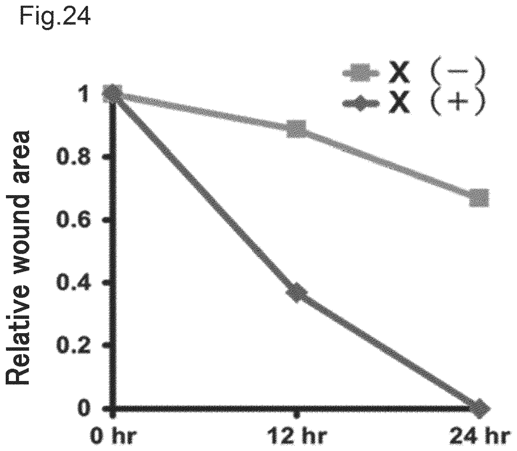

Here, in each image, a scale bar of 100 µm is shown. In each image shown in Fig. 23, for convenience of illustration, the boundary of the cell scratch portion at the time of cell scratching (at 0 hour of culture after cell scratching) is indicated by thick lines, and the boundary of the cells having migrated during the subsequent culture is indicated by thin lines. Fig. 24 is a graph illustrating the cell migration ability and proliferation potential of iBPCs. The ordinate in Fig. 24 indicates a relative value when the distance of the cell scratch portion at the time of cell scratching (0 hour of culture after cell scratching) is set to 1 as a reference.

The results shown in Fig. 23 and 24 have revealed the following. In iBPCs (X (+)) induced to differentiate by adding A 83-01, the distance of the scratch portion was shortened as the culture time was elapsed after cell scratching when compared to iBPCs (X (-)) induced to differentiate without adding A 83-01. In particular, 24 hours after cell scratching, the distance was 0. As such, addition of A 83-01 to the culture medium for inducing differentiation into iBPCs was found to be able to enhance the cell migration ability and proliferation potential of iBPCs. This has demonstrated that by inducing differentiation from pluripotent stem cells to brain pericytes while using a medium containing A 83-01, the cells can further mimic (can be more similar to) brain pericytes *in vivo.*

### Industrial Applicability

The present disclosure makes it possible to produce brain microvascular endothelial-like cells more similar to brain microvascular endothelial cells *in vivo.* In addition, the present disclosure can facilitate production of brain microvascular endothelial-like cells since frozen vascular endothelial progenitor cells can be used as a differentiation source. This enables brain microvascular endothelial-like cells to be stably supplied, contributing to the establishment of a supply system of brain microvascular endothelial-like cells for pharmaceutical companies and research institutions. Furthermore, the BBB model constructed using the present disclosure can be used for, for example, a system for evaluating efficacy/safety of some pharmaceutical product.

The present invention is not at all limited to the description of embodiments and examples of the invention. Various modifications that can be easily conceived by those skilled in the art without departing from the scope of the claims are also included in the present invention. The contents of the research articles, published patent application publications, patent publications, and others specified herein are incorporated by reference in their entirety.

### [Sequence Listing Free Text]

SEQ ID NO: 1: Description of synthetic sequence: CDH5 forward primer
SEQ ID NO: 2: Description of synthetic sequence: CDH5 reverse primer
SEQ ID NO: 3: Description of synthetic sequence: MDR1 forward primer
SEQ ID NO: 4: Description of synthetic sequence: MDR1 reverse primer
SEQ ID NO: 5: Description of synthetic sequence: BCRP forward primer
SEQ ID NO: 6: Description of synthetic sequence: BCRP reverse primer
SEQ ID NO: 7: Description of synthetic sequence: GLUT1 forward primer
SEQ ID NO: 8: Description of synthetic sequence: GLUT1 reverse primer
SEQ ID NO: 9: Description of synthetic sequence: Occludin forward primer
SEQ ID NO: 10: Description of synthetic sequence: Occludin reverse primer
SEQ ID NO: 11: Description of synthetic sequence: ZO-1 forward primer
SEQ ID NO: 12: Description of synthetic sequence: ZO-1 reverse primer
SEQ ID NO: 13: Description of synthetic sequence: LAT1 forward primer
SEQ ID NO: 14: Description of synthetic sequence: LAT1 reverse primer
SEQ ID NO: 15: Description of synthetic sequence: HPRT1 forward primer
SEQ ID NO: 16: Description of synthetic sequence: HPRT1 reverse primer
SEQ ID NO: 17: Description of synthetic sequence: PECAM1 forward primer
SEQ ID NO: 18: Description of synthetic sequence: PECAM1 reverse primer
SEQ ID NO: 19: Description of synthetic sequence: PDGFR-β forward primer
SEQ ID NO: 20: Description of synthetic sequence: PDGFR-β reverse primer
SEQ ID NO: 21: Description of synthetic sequence: NG2 forward primer
SEQ ID NO: 22: Description of synthetic sequence: NG2 reverse primer
SEQ ID NO: 23: Description of synthetic sequence: α-SMA forward primer
SEQ ID NO: 24: Description of synthetic sequence: α-SMA reverse primer

## Claims

1. A method of producing brain microvascular endothelial-like cells, comprising a culturing step of culturing vascular endothelial progenitor cells by using a Laminin511 fragment, Fibronectin, and Collagen type IV.

2. The method of producing brain microvascular endothelial-like cells according to claim 1, wherein the culturing step comprises culturing using a medium containing B27 (registered trademark) supplement, A 83-01, and Fibroblast Growth Factor-2 (FGF2).

3. The method of producing brain microvascular endothelial-like cells according to claim 1 or 2, wherein the vascular endothelial progenitor cells are cells differentiated from pluripotent stem cells.

4. The method of producing brain microvascular endothelial-like cells according to claim 3, wherein the vascular endothelial progenitor cells are cells differentiated from human induced pluripotent stem cells.

5. The method of producing brain microvascular endothelial-like cells according to claim 3 or 4, wherein the vascular endothelial progenitor cells are cells differentiated using vascular endothelial growth factor.

6. The method of producing brain microvascular endothelial-like cells according to any one of claims 1 to 5, wherein the culturing step comprises co-culturing the vascular endothelial progenitor cells with brain pericytes.

7. The method of producing brain microvascular endothelial-like cells according to claim 6, wherein the brain pericytes are cells differentiated, using an A 83-01-containing medium, from pluripotent stem cells.

8. A method for evaluating permeability of a test substance across a blood-brain barrier, comprising using a cell layer of brain microvascular endothelial-like cells obtained by the production method according to any one of claims 1 to 7.

9. The method according to claim 8, comprising the following steps (i) to (iii):
(i) preparing the cell layer;
(ii) bringing the test substance into contact with the cell layer; and
(iii) evaluating permeability of the test substance across the blood-brain barrier by quantifying the test substance having permeated the cell layer.

10. A method for evaluating an effect of a test substance on a blood-brain barrier function, comprising using a cell layer of brain microvascular endothelial-like cells obtained by the production method according to any one of claims 1 to 7.

11. Brain microvascular endothelial-like cells obtained by the production method according to any one of claims 1 to 7.

12. The brain microvascular endothelial-like cells according to claim 11, wherein an expression level of PECAM1 is higher than that in primary cultured brain microvascular endothelial cells and immortalized brain microvascular endothelial cells, and a TEER value is 50 Ω × cm² or higher.

13. A method of inducing differentiation of brain microvascular endothelial-like cells, comprising a culturing step of culturing vascular endothelial progenitor cells by using Laminin511 fragment, Fibronectin, and Collagen type IV.
